(19) Europäisches Patentamt
European Patent Office
Office européen des brevets

(11) Veröffentlichungsnummer: **0 600 967 B1**

(12) **EUROPÄISCHE PATENTSCHRIFT**

(45) Veröffentlichungstag der Patentschrift: **19.04.95**

(51) Int. Cl.$^6$: **C07D 521/00**, A01N 47/36, C07D 239/47, C07D 239/48, C07D 239/52

(21) Anmeldenummer: **92917618.8**

(22) Anmeldetag: **25.08.92**

(86) Internationale Anmeldenummer: **PCT/EP92/01879**

(87) Internationale Veröffentlichungsnummer: **WO 93/05048 (18.03.93 93/08)**

Die Akte enthält technische Angaben, die nach dem Eingang der Anmeldung eingereicht wurden und die nicht in dieser Patentschrift enthalten sind.

# (54) HERBIZIDE SULFONYLHARNSTOFFE, VERFAHREN UND ZWISCHENPRODUKTE ZU IHRER HERSTELLUNG.

(30) Priorität: **28.08.91 DE 4128441**

(43) Veröffentlichungstag der Anmeldung: **15.06.94 Patentblatt 94/24**

(45) Bekanntmachung des Hinweises auf die Patenterteilung: **19.04.95 Patentblatt 95/16**

(84) Benannte Vertragsstaaten: **AT BE CH DE ES FR GB IT LI NL**

(56) Entgegenhaltungen:
**EP-A- 0 169 815**
**EP-A- 0 446 743**

**Tetrahedron Letters, 22 (1981), S. 323-326**

(73) Patentinhaber: **BASF Aktiengesellschaft**
**Carl-Bosch-Strasse 38**
**D-67063 Ludwigshafen (DE)**

(72) Erfinder: **HAMPRECHT, Gerhard**
**Rote-Turm-Strasse 28**
**D-6940 Weinheim (DE)**
Erfinder: **MAYER, Horst**
**Faselwiese 19**
**D-6700 Ludwigshafen (DE)**
Erfinder: **WESTPHALEN, Karl-Otto**
**Mausbergweg 58**
**D-6720 Speyer (DE)**
Erfinder: **WALTER, Helmut**
**Gruenstadter Strasse 82**
**D-6719 Obrigheim (DE)**
Erfinder: **GERBER, Matthias**
**Ritterstrasse 3**
**D-6704 Mutterstadt (DE)**
Erfinder: **KARDORFF, Uwe**
**D 3, 4**
**D-6800 Mannheim 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft substituierte Sulfonylharnstoffe der allgemeinen Formel I,

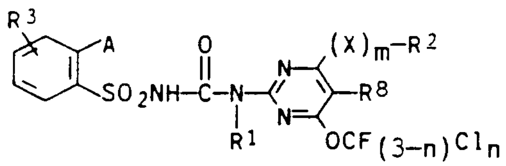

I

in der n und m für 0 oder 1 stehen und die Substituenten folgende Bedeutung haben:

- $R^1$ Wasserstoff, $C_1$-$C_4$-Alkyl, $C_3$-$C_6$-Alkenyl oder $C_3$-$C_6$-Alkinyl;
- $R^2$ Halogen oder Trifluormethyl, wenn m für 0 steht oder $C_1$-$C_4$-Alkyl, $C_3$-$C_6$-Alkenyl oder $C_3$-$C_6$-Alkinyl, wenn m 1 bedeutet oder Trifluor- oder Chlordifluormethyl, wenn X für 0 oder S und m für 1 stehen;
- X O, S oder N-$R^4$, wobei $R^4$ für Wasserstoff oder $C_1$-$C_4$-Alkyl steht ;
- $R^3$ Wasserstoff, Halogen, $C_1$-$C_4$-Alkyl, $C_1$-$C_4$-Halogenalkyl, $C_1$-$C_4$-Alkoxy oder $C_1$-$C_4$-Halogenalkoxy;
- A $NO_2$, $NH_2$, OH, CN, SCN, $S(O)_oR^5$, $SO_2NR^6R^7$, eine Gruppe $ER^7$, in der E für O, S oder $NR^9$ steht,

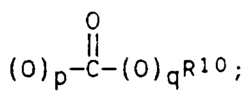

  $CH=NOCH_3$; $C_1$-$C_4$-Alkyl, unsubstituiert oder 1- bis 3-fach substituiert durch Methoxy, Ethoxy, $SO_2CH_3$, Cyano, Rhodano, $SCH_3$; $C_2$-$C_4$-Alkenyl, unsubstituiert oder 1- bis 3-fach substituiert durch Halogen, Nitro oder Cyano;
- $R^5$ eine $C_1$-$C_6$-Alkylgruppe, welche einen bis drei der folgenden Reste tragen kann: Halogen, $C_1$-$C_4$-Alkoxy, $C_1$-$C_4$-Halogenalkoxy, $C_1$-$C_4$-Alkoxy-$C_1$-$C_2$-alkoxy, $C_3$-$C_7$-Cycloalkyl und/oder Phenyl; eine $C_5$-$C_7$-Cycloalkylgruppe, welche ein bis drei $C_1$-$C_4$-Alkylgruppen tragen kann; eine $C_2$-$C_6$-Alkenylgruppe oder eine $C_3$-$C_6$-Alkinylgruppe;
- $R^6$ Wasserstoff, eine $C_1$-$C_2$-Alkoxygruppe, eine $C_1$-$C_6$-Alkylgruppe, oder gemeinsam mit $R^7$ eine $C_4$-$C_6$-Alkylenkette, worin eine Methylengruppe durch ein Sauerstoffatom oder eine $C_1$-$C_4$-Alkyliminogruppe ersetzt sein kann;
- $R^7$ $C_1$-$C_4$-Alkyl, $C_2$-$C_4$-Alkenyl oder $C_3$-$C_4$-Alkinyl, wobei die genannten Reste noch ein bis vier Halogen- oder $C_1$-$C_4$-Alkoxyreste tragen können; eine $C_3$-$C_6$-Cycloalkylgruppe; ferner im Fall von E = $NR^9$ noch Methylsulfon, Trifluormethylsulfon, Ethylsulfon, Acetyl, das ein bis drei Halogenatome tragen kann, Methoxycarbonyl, Dimethylcarbamoyl oder Dimethylsulfamoyl;
- o 0, 1 oder 2;
- p,q 0 und/oder 1, wobei im Falle von p=0 q ebenfalls für 0 steht;
- $R^8$ Wasserstoff oder Halogen;
- $R^9$ Wasserstoff, Methyl oder Ethyl;
- $R^{10}$ $C_1$-$C_3$-Alkyl, $C_1$-$C_3$-Halogenalkyl, $C_1$-$C_2$-Alkoxy-$C_1$-C2-alkyl, $C_2$-$C_4$-Alkenyl, $C_3$-$C_6$-Cycloalkyl, $C_2$-$C_3$-Halogenalkenyl, ferner im Fall von p = 1 und q = 0 noch $C_1$-$C_3$-Alkylamino oder Di-($C_1$-$C_2$-Alkyl)amino

sowie deren umweltverträgliche Salze.

Des weiteren betrifft die Erfindung ein Verfahren zur Herstellung der Verbindungen I sowie ihre Verwendung als herbizide Mittel und Zwischenprodukte zur Herstellung der Sulfonylharnstoffe I.

Aus der US-A-4 547 215 sind verschiedene Sulfonylharnstoffe als Herbizide bekannt, welche im Pyrimidinteil durch Chlor substituiert sind. In den EP-A-72 347, 84 020 und 169 815 werden Sulfonylharnstoffe beschrieben, die im Pyrimidinteil durch den Difluormethoxy- bzw. den Bromdifluormethoxyrest substituiert sind. Diese Verbindungen lassen jedoch wegen der unbefriedigenden Selektivität gegen Schadpflanzen zu wünschen übrig. Sulfonylharnstoffe mit unterschiedlichem Substitutionsmuster am Phenylrest werden in den älteren Anmeldungen EP-A-446 743 und EP-A-469 460 offenbart.

Der Erfindung lagen neue Verbindungen aus der Klasse der Sulfonylpyrimidyl-harnstoffe mit verbesserten herbiziden Eigenschaften als Aufgabe zugrunde. Entsprechend dieser Aufgabe wurden die eingangs

definierten Sulfonylharnstoffe gefunden.

Ferner wurde gefunden, daß die Verbindungen der Formel I sowie deren Alkali- und Erdalkalimetallsalze eine gute Selektivität gegen Schadpflanzen in Kulturen wie Getreide und Mais haben.

Außerdem wurden chemisch eigenartige Verfahren zur Herstellung der Verbindungen I gefunden. Im Vergleich zu dem Stand der Technik lassen sich die Sulfonylharnstoffe I entgegen der Erwartung regioselektiv und in hoher Ausbeute und Reinheit herstellen, wenn man ausgeht von substituierten 2-Amino-4-fluoralkoxy-6-pyrimidinen der allgemeinen Formel IIIa

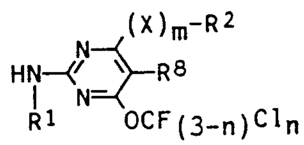

IIIa

in der m für 1 und n für O oder 1 stehen und die Substituenten folgende Bedeutung haben:

- $R^1$ Wasserstoff, $C_1$-$C_4$-Alkyl, $C_3$-$C_6$-Alkenyl oder $C_3$-$C_6$-Alkinyl;
- $R^2$ $C_1$-$C_4$-Alkyl, $C_3$-$C_6$-Alkenyl oder $C_3$-$C_6$-Alkinyl;
- $R^8$ Wasserstoff oder Halogen;
- X O, S oder N-$R^4$, wobei
- $R^4$ für Wasserstoff oder $C_1$-$C_4$-Alkyl steht.

Zur Herstellung von im Pyrimidinteil halogensubstituierten Verbindungen ($R^2$ = Hal, m = 0) geht man von entsprechend substituierten 2-Amino-4-fluoralkoxy-6-halogen-pyrimidinen der Struktur IIIb aus (s. Formelschema 2), deren Herstellung Gegenstand der deutschen Anmeldung P 40 07 316 (O.Z. 0050/41451) ist. Pyrimidinzwischenprodukte mit m = 0 und $R^2$ = Trifluormethyl erhält man in analoger Weise gemäß Formelschema 3.

Die erfindungsgemäßen Sulfonylharnstoffe der Formel I sind auf den in Formelschema 1 beschriebenen Wegen A, B und C zugänglich:

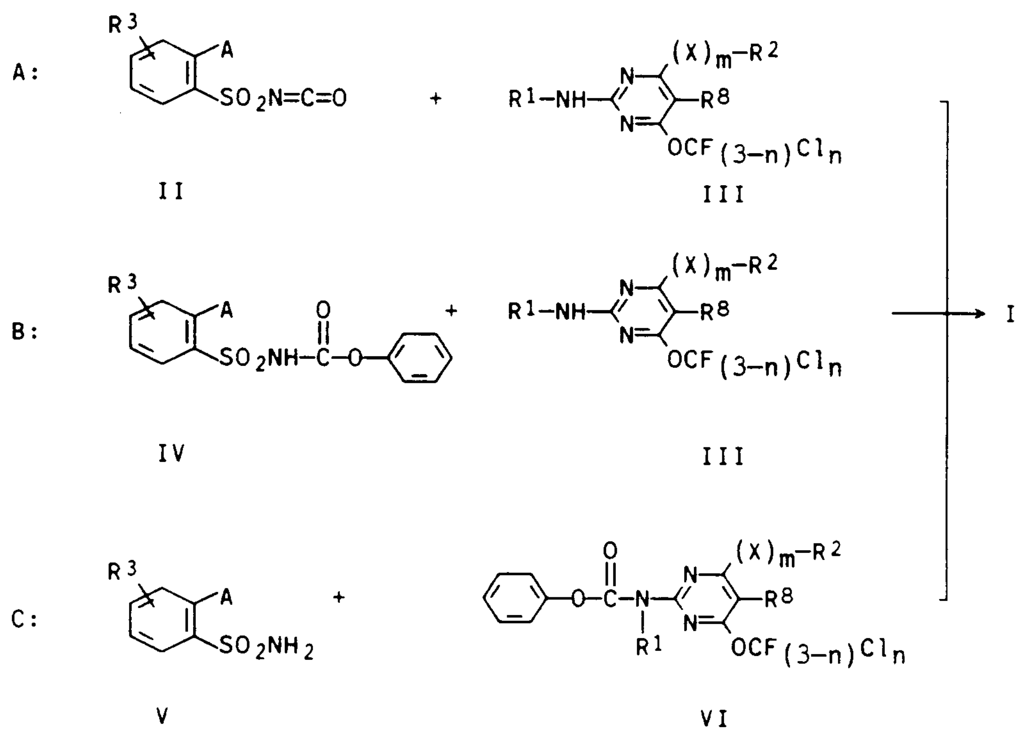

A: II + III → I

B: IV + III → I

C: V + VI → I

Ausführungsform A

Man setzt ein Sulfonylisocyanat II in an sich bekannter Weise (EP-A-162 723) in einem inerten organischen Lösungsmittel mit ungefähr der stöchiometrischen Menge eines 2-Aminopyrimidinderivats III

bei einer Temperatur von 0 bis 120 °C, vorzugsweise 10 bis 100 °C um. Die Reaktion kann drucklos oder unter Druck (bis 50 bar), vorzugsweise bei 1 bis 5 bar, kontinuierlich oder diskontinuierlich durchgeführt werden. Geeignete Lösungsmittel sind in der obengenannten Literatur aufgeführt.

Ausführungsform B:

Man setzt ein entsprechendes Sulfonylcarbamat der Formel IV in an sich bekannter Weise (EP-A-162 723) in einem inerten organischen Lösungsmittel bei einer Temperatur zwischen 0 und 120 °C, vorzugsweise 10 bis 100 °C mit einem 2-Aminopyrimidinderivat um. Es können hierbei Basen wie tertiäre Amine zugesetzt werden, wodurch die Reaktion beschleunigt und die Produktqualität verbessert werden.

Geeignete Basen hierfür sind z. B. tertiäre Amine wie Pyridin, die Picoline, 2,4- und 2,6-Lutidin, 2,4,6-Collidin, p-Dimethylaminopyridin, 1,4-Diaza(2,2,2)bicyclooctan (DABCO) und 1,8-Diazabicyclo(5,4,0)-undec-7-en.

Zweckmäßig verwendet man als Lösungsmittel die in der Literatur angegebenen und/oder Halogenkohlenwasserstoffe wie Dichlormethan und Chlorbenzol, Ether wie Diethylether, Tetrahydrofuran und Dioxan, Acetonitril, Dimethylformamid und/oder Essigsäureethylester in einer Menge von 100 bis 4000 Gew.%, vorzugsweise von 1000 bis 2000 Gew.%, bezogen auf die Ausgangsstoffe II, IV und V.

Im Rahmen der Herstellung der erfindungsgemäßen Verbindungen sind die 2-Aminopyrimidin-Zwischenprodukte III auf folgende vorteilhafte Weise zugänglich:

Formelschema 2

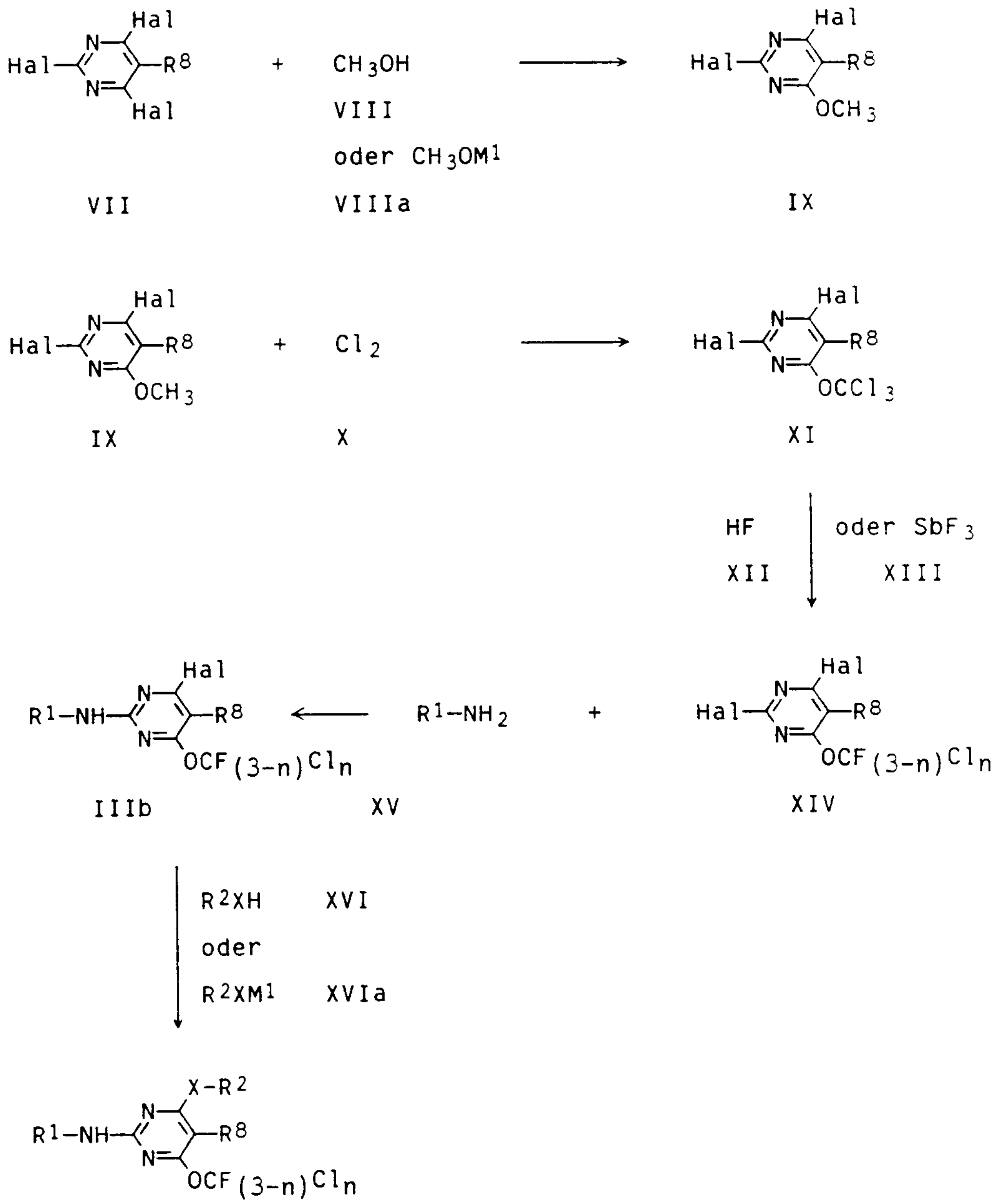


VII

IX

XI

XIV

IIIb

IIIa

In entsprechender Weise gelangt man zu den 2-Amino-6-trifluormethylpyrimidinderivaten IIIc, wenn man anstelle der 2,4,6-Trihalogenverbindungen VII die entsprechenden 2,4-Dihalogen-6-trichlormethylpyrimidine gemäß Formelschema 3 umsetzt (s. Beispiele I.1, I.6 und I.12).

Formelschema 3

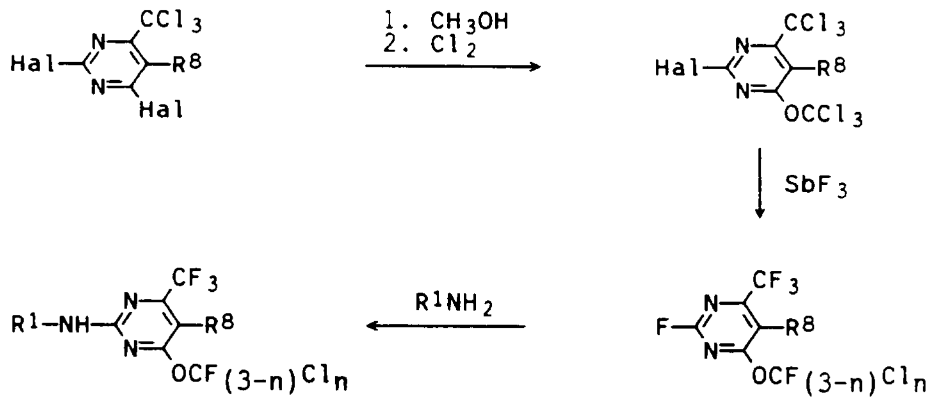


IIIc

Ausgehend von den Zwischenprodukten XIV in Formelschema 2 und Substitution des Halogenatoms in 4-Position durch die in Formelschema 3 beschriebene Reaktionssequenz (1. $CH_3OH$, 2. $Cl_2$, 3. $SbF_3$) sowie anschließende Umsetzung mit $R^1NH_2$ gelangt man zu den Zwischenprodukten IIId

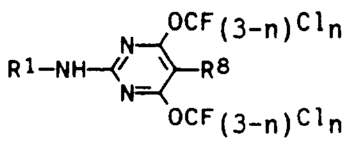

IIId

Entsprechend Formelschema 2 kann man beispielsweise ein 2,4,6-Trihalogenpyrimidin VII, bekannt aus J.Med.Chem. 6, 688 (1963) oder käuflich zu erwerben, in einem aprotisch polaren Lösungsmittel

a) mit Methanol VIII in Anwesenheit oder Abwesenheit einer Base oder

b) mit einem Methanolat VIIIa in Gegenwart von Methanol VIII bei Temperaturen zwischen -40 und 120 °C zum Methoxypyrimidin IX umsetzen. Diese Reaktionen können drucklos oder unter Druck (1 bis 10 bar, vorzugsweise 1 bis 5 bar), kontinuierlich oder diskontinuierlich durchgeführt werden.

Hal in Formel VII steht für Fluor, Chlor oder Brom.

$M^1$ in Formel VIIIa bedeutet ein Alkalimetallkation wie Lithium-, Natrium-und Kaliumkation oder das Equivalent eines Erdalkalimetallkations wie Magnesium-, Calcium- und Bariumkation.

Für die Umsetzung des Trihalogenpyrimidins mit Methanol VIII sind folgende Lösungsmittel geeignet:

Ether wie Methyl-tert.-butylether, Diethylether, Ethylpropylether, n-Butylethylether, Di-n-butylether, Diisobutylether, Diisoamylether, Di-isoopropylether, Cyclohexylmethylether, Tetrahydrofuran, 1,2-Dimethoxyethan, Diethylenglycoldimethylether und Anisol, chlorierte Kohlenwasserstoffe wie 1,1,2,2-Tetrachlorethan, 1,1-Dichlorethylen, Chlorbenzol, 1,2-Dichlorbenzol und 1-Chlornaphthalin sowie entsprechende Gemische.

Zweckmäßig verwendet man das Lösungsmittel in einer Menge von 100 bis 2000 Gew.%, vorzugsweise 500 bis 1500 Gew.%, bezogen auf den Ausgangsstoff VII.

Zweckmäßig führt man jedoch die Umsetzung der Ausgangsstoffe VII mit VIII direkt in überschüssigem Methanol VIII als Lösungsmittel durch. Gegebenenfalls gibt man ein Alkalimethanolat VIIIa in einer equivalenten Menge oder einem über- oder Unterschuß von bis zu 5 %, bezogen auf den Ausgangsstoff VII, zu einer Suspension des Ausgangsstoffes VII in der 5-bis 20-fachen Gewichtsmenge Alkohol VIII als Lösungsmittel, bezogen auf den Ausgangsstoff VII, innerhalb bis zu einer Stunde bei einer Temperatur von -20 bis 80 °C. Zur Beendigug der Umsetzung rührt man dann noch 1/2 bis 8 Stunden bei 0 bis 120 °C, vorzugsweise 0 bis 100 °C nach.

Zur Isolierung der Methoxy-pyrimidine dienen die hierfür in der Literatur üblichen Aufarbeitungsmethoden wie destillative oder chromatographische Aufarbeitung.

Die Chlorierung des Methoxy-pyrimidins IX mit Chlor X zu dem Trichlormethoxy-pyrimidin XI führt man beispielsweise bei einer Temperatur von 60 bis 180 °C durch.

Als Chlorierungsmittel sind elementares Chlor oder Chlor abgebende Substanzen wie Sulfurylchlorid oder Phosphorpentachlorid geeignet. Chlor kann dabei auch in situ durch Oxydation von Chlorwasserstoffsäure, beispielsweise mit Braunstein oder Wasserstoffperoxid oder durch anodische Chlorierung hergestellt werden.

Die Umsetzung kann in Gegenwart eines inerten Lösungsmittels, beispielsweise einem chlorierten Kohlenwasserstoff wie Chloroform, Tetrachlorkohlenstoff, Chlorbenzol, 1,2-, 1,3- oder 1,4-Dichlorbenzol, einem Nitril wie Acetonitril, Propionitril, einer Nitroverbindung wie Nitrobenzol, einer Carbonsäure wie Essigsäure, Propionsäure, einem Säureanhydrid wie Essigsäureanhydrid, einem Säurechlorid wie Chloracetylchlorid, α-Chlorpropionsäurechlorid, α,α-Dichlorpropionsäurechlorid, einem anorganischen Säurehalogenid wie Phosphortrichlorid oder Phosphoroxychlorid oder bevorzugt ohne Lösungsmittel in der Schmelze des Ausgangsstoffes IX durchgeführt werden.

Gegebenenfalls kann man die Reaktion durch Zugabe eines Radikalstarters beschleunigen; als solcher eignet sich die Bestrahlung mit Licht, vorzugsweise UV-Licht oder die Zugabe von α,α'-Azoisobutyronitril, zweckmäßig in einer Menge von 0,2 bis 7 mol%, bezogen auf den Ausgangsstoff IX. Man kann die Reaktion auch durch Zugabe eines Katalysators beschleunigen; als solcher eignet sich Phosphorpentachlorid, zweckmäßig in einer Menge von 0,5 bis 7 mol% bezogen auf den Ausgangsstoff IX. In diesem Fall legt man den Ausgangsstoff IX zusammen mit dem Katalysator vor und beginnt dann mit der Chlorierung. Statt des Phosphorpentachlorids kann man auch eine dieses unter den Reaktionsbedingungen bildende Ausgangskomponente, z.B. Phosphortrichlorid oder gelben Phosphor, zugeben und dann mit der Chlorierung beginnen.

Ausgangsstoff IX kann mit Chlor in annähernd stöchiometrischer Menge oder vorzugsweise im Überschuß, vorteilhaft mit 3,1 bis 11, insbesondere 3,3 bis 5 mol $Cl_2$ je Equivalent Methoxy in dem Ausgangsstoff IX umgesetzt werden. Die Umsetzung kann bei einer Temperatur von 60 bis 180 ° C, vorteilhaft von 100 bis 150 ° C, drucklos oder unter Druck kontinuierlich oder diskontinuierlich durchgefürt werden.

Chloriert man bei 1 bar, so werden zweckmäßig 3,3 bis 5 mol Chlorgas, bezogen auf ein Equivalent Methoxy in dem Ausgangsstoff IX, eingesetzt, was einem Chlorumsatz von 91 bis 60 % entspricht. Durch geeignete apparative Maßnahmen, z.B. durch Anwendung von mäßigem Überdruck, zweckmäßig 1 bis 10 bar, oder durch Verwendung einer Blasensäule, läßt sich der Chlorumsatz erhöhen. Vorteilhaft läßt man das Chlorgas möglichst lange mit der organischen Phase in Berührung kommen, indem diese beispielsweise stark gerührt wird oder das Chlorgas eine dicke Schicht der organischen Phase durchdringen muß.

Die Reaktionszeit beträgt im allgemeinen etwa 0,5 bis 12 Stunden.

In einer bevorzugten Ausführungsform des Verfahrens geht man so vor, daß man innerhalb von 0,5 bis 12 Stunden, vorzugsweise 1 bis 10 Stunden die erforderliche Menge Chlorgas unter intensivem Rühren in den flüssigen Ausgangsstoff IX einleitet, wobei man zunächst bei einer Temperatur von 60 bis 80 ° C beginnt und diese - gegebenenfalls unter Ausnutzung des exothermen Charakters der Reaktion - kontinuierlich steigert, so daß gegen Ende die Umsetzung bei einer Temperatur von 100 bis 150 ° C durchgeführt wird. Bei größeren Reaktionsansätzen trägt man dem exothermen Charakter durch äußere Kühlung bzw. geeignete Dosierung der Chlormenge Rechnung; mit dem Abflauen der Reaktion entfernt man das Kältebad und kann gegebenenfalls noch nacherhitzen.

Die Aufarbeitung und Isolierung der Endstoffe kann in üblicher Weise erfolgen. Beispielsweise kann man aus der heißen organischen Phase mittels eines Inertgases Reste an Chlorwasserstoff, Chlor oder Katalysator austragen; dabei hinterbleibt in hoher Ausbeute ein bereits recht reines Rohprodukt. Durch Destillation oder Chromatographie kann es weiter gereinigt oder aber direkt für weitere Umsetzungen eingesetzt werden.

Die Umsetzung des Trichlormethoxy-pyrimidins XI mit einem Halogenaustauschmittel führt man beispielsweise bei einer Temperatur von 0 bis 170 ° C durch.

Als Halogenaustauschmittel sind Antimontrifluorid in Gegenwart oder Abwesenheit katalytischer Mengen eines Antimon(V)salzes, z.B. Antimon(V)chlorid oder Fluorwasserstoff geeignet.

Zweckmäßig verwendet man einen Überschuß von 1 bis 200, vorzugsweise 5 bis 20 mol% Antimontrifluorid pro Trichlormethylequivalent. Die Katalysatormenge an Antimon(V)salz beträgt zwischen 1 bis 20, vorzugsweise 5 bis 18 mol% pro Trichlormethylequivalent. Vorzugsweise dosiert man den Ausgangsstoff XI bei 90 bis 130 ° C zu der Mischung des Halogenaustauschmittels und erwärmt dann noch zwischen 10 und ca. 120 Minuten auf eine Temperatur zwischen 140 bis 170 ° C. Anschließend wird durch Destillation aufgearbeitet.

Man kann die Reaktion jedoch auch kontinuierlich führen, den Ausgangsstoff XI bei 140 bis 170 ° C innerhalb 10 bis ca. 120 Minuten zugeben und gleichzeitig unter vermindertem Druck den entstehenden niedriger siedenden Endstoff XIV abdestillieren. Spuren mitgerissender Antimonsalze lassen sich durch Extraktion mit konz. Salzsäure beseitigen.

Arbeitet man ohne Antimon(V)salz-Katalyse oder setzt nur geringe Mengen, z. B. 0,2 bis 1 mol% ein, und reduziert die Menge an Antimontrifluorid auf 60 bis 90 mol% pro Trichlormethylequivalent, so bleibt der Halogenaustausch auf der Chlordifluormethoxystufe stehen.

Anstelle von Antimontrifluorid kann der Halogenaustausch auch mit Fluorwasserstoff bei 0 bis 170°C, vorzugsweise 40 bis 120°C, durchgeführt werden. Hierzu versetzt man in einem Autoklaven den Ausgangsstoff XI mit einem Überschuß von 300 bis 700, vorzugsweise 350 bis 400 mol% Fluorwasserstoff pro Trichlormethylequivalent und rührt 10 Minuten bis ca. 10 Stunden. Nach dem Entspannen und der Entfernung flüchtiger Bestandteile wird wie beschrieben aufgearbeitet.

Die Umsetzung des Fluormethoxy-pyrimidins XIV mit einem Amin XV führt man beispielsweise bei einer Temperatur von -80 bis 40°C durch.

In Formel XV steht $R^1$ beispielsweise für Wasserstoff, $C_1$-$C_4$-Alkyl wie Methyl, Ethyl, n-Propyl, i-Propyl, n-Butyl, sek.-Butyl, i-Butyl, tert.-Butyl ; $C_3$-$C_4$-Alkenyl wie 2-Propenyl, 2-Methylethenyl, 2-Butenyl, 3-Butenyl, 1-Methyl-2-propenyl oder 2-Methyl-2-propenyl ; $C_3$-$C_4$-Alkinyl wie Propargyl, 2-Butinyl, 3-Butinyl oder 1-Methyl-2-propinyl.

Unter den Aminen, welche eingesetzt werden können, sollen die folgenden erwähnt werden: Ammoniak, Methylamin, Ethylamin, n-Propylamin, Isopropylamin, n-Butylamin, Isobutylamin, sek.-Butylamin, tert.-Butylamin, 2-Propenylamin, 2-Methylethenylamin, 2-Butenylamin, 3-Butenylamin, 1-Methyl-2--propenylamin, 2-Methyl-2-propenylamin, Propargylamin, 2-Butinylamin, 3-Butinylamin und 1-Methyl-2-propinylamin.

Die 2,6-Dihalogen-pyrimidine XIV können in einem aprotisch polaren Lösungsmittel mit den Aminen XV bei einer Temperatur von -80 bis 40°C umgesetzt werden, wobei man das Amin XV entweder in einem Überschuß einsetzt oder eine organische Hilfsbase verwendet.

Für die Umsetzung des 2,6-Dihalogen-pyrimidins XIV mit dem Amin XV sind beispielsweise folgende Lösungsmittel geeignet:

Ether wie Methyl-tert.-butylether, Diethylether, Ethylpropylether, n-Butylethylether, Di-n-butylether, Diisobutylether, Diisoamylether, Diisopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat sowie chlorierte Kohlenwasserstoffe wie Methylenchlorid, 1,1,2,2-Tetrachlorethan, 1,1-Dichlorethylen, 1,2-Dichlorethan, Chlorbenzol, 1,2-Dichlorbenzol und 1-Chlornaphthalin und Gemische dieser Lösungsmittel.

Zweckmäßig verwendet man das Lösungsmittel in einer Menge von 100 bis 2000 Gew.%, vorzugsweise 400 bis 1200 Gew.%, bezogen auf den Ausgangsstoff XIV.

Vorteilhaft gibt man 1,8 bis 2,5, insbesondere 1,95 bis 2,2 mol-Equivalent des Amins XV, bezogen auf den Ausgangsstoff XIV innerhalb 0,5 bis 2 Stunden zu einer Mischung von Ausgangsstoff XIV in einem der vorgenannten Lösungsmittel bei (-80) bis 40°C, vorzugsweise -70 bis 25°C, rührt bis zur Vervollständigung der Reaktion bis zu 3 Stunden nach und läßt dann zur Aufarbeitung auf 25°C erwärmen.

Setzt man nur ungefähr stöchiometrische Mengen des Amins XV ein, so verwendet man zweckmäßig 0,9 bis 1,1 Equivalente einer organischen Hilfsbase, bezogen auf den Ausgangsstoff XIV. Als Hilfsbase eignen sich organische Basen wie Trimethylamin, Triethylamin, N-Ethyl-diisopropylamin, Triisopropylamin, N,N-Dimethylanilin, N,N-Dimethylcyclohexylamin, N-Methylpyrrolidin, Pyridin, Chinolin, α-,β-, γ-Picolin, 2,4- und 2,6-Lutidin und Triethylendiamin.

Die Reaktion kann drucklos oder unter Druck kontinuierlich oder diskontinuierlich durchgeführt werden.

Zur Aufarbeitung extrahiert man das Umsetzungsgemisch mit Wasser zur Entfernung der Salze, trocknet und reinigt die organische Phase, z. B. durch Chromatographie. Man kann jedoch auch direkt die organische Phase einengen und den Rückstand mit einem Lösungsmittel verrühren.

Die erfindungsgemäßen 2-Amino-4-fluoralkoxy-pyrimidine der Formel IIIa erhält man vorteilhaft in der Weise, daß man 2-Amino-4-fluoralkoxy-6--halogen-pyrimidine der Formel IIIb

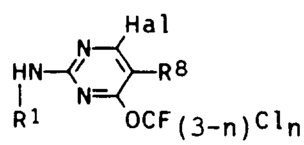

IIIb

in der Hal für Fluor, Chlor oder Brom steht und $R^1$, $R^8$ und n die vorgenannte Bedeutung haben, mit einem Nucleophil der Formel XVI,

$H$-$X$-$R^2$ XVI

in der X und $R^2$ die vorgenannte Bedeutung besitzen, oder dessen Salz umsetzt.

Die Umsetzung kann für den Fall der Verwendung von 2-Amino-4-fluor-6-trifluormethoxypyrimidin und Methylamin durch folgendes Schema beschrieben werden:

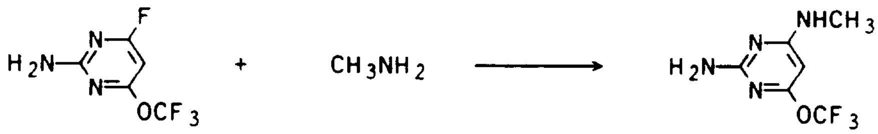

Für den Fall der Verwendung von 2-Amino-4-fluor-6-chlordifluormethoxypyrimidin und Natriummethylat läßt sich die Umsetzung durch folgendes Schema wiedergeben:

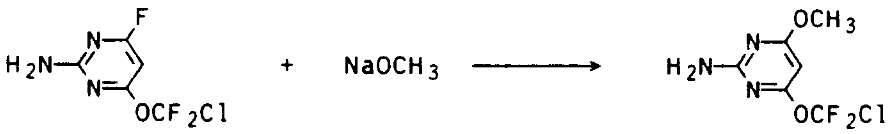

Das Verfahren liefert auf einfachem und wirtschaftlichem Weg neue 2-Amino-4-fluoralkoxy-pyrimidine in hoher Ausbeute und Reinheit. Entgegen der Erwartung werden Fluoralkoxygruppen nicht substituiert. Auch das in der Etherseitenkette stehende Chloratom bleibt trotz der alkalischen Reaktionsbedingungen erhalten. Im Hinblick auf den Stand der Technik (s. z.B. EP-A-70 804) sind alle diese vorteilhaften Eigenschaften überraschend.

Bevorzugte Zwischenprodukte IIIa und dementsprechend bevorzugte Ausgangsstoffe IIIb sind solche, in deren Formeln $R^1$ und $R^2$ $C_1$-$C_4$-Alkyl wie Methyl, Ethyl, n-Propyl, i-Propyl, n-Butyl, sek.-Butyl, i-Butyl, tert.-Butyl; $C_3$-$C_4$-Alkenyl wie 2-Propenyl, 2-Methylethenyl, 2-Butenyl, 3-Butenyl, 1-Methyl-2-propenyl, 2-Methyl-2-propenyl ; $C_3$-$C_4$-Alkinyl wie Propargyl, 2-Butinyl, 3-Butinyl, 1-Methyl-2-propinyl, darüberhinaus kann $R^1$ auch Wasserstoff bedeuten,

- X O, S oder N-$R^4$, wobei
- $R^4$ für Wasserstoff, Methyl, Ethyl, n-Propyl, i-Propyl, n-Butyl, sek.-Butyl, i-Butyl oder tert.-Butyl steht,
- $R^8$ Wasserstoff und
- n 0 oder 1

bedeuten.

Die Umsetzung des 2-Amino-4-fluoralkoxy-pyrimidins IIIb mit einem Nucleophil XVI oder dessen Salz XVIa führt man beispielsweise bei einer Temperatur von -80 bis 80 °C durch. Als Nucleophile XVI sind Ammoniak, aliphatische Amine, Alkohole und Thiole geeignet.

Unter den Aminen, welche als Nucleophile eingesetzt werden können, sollen die folgenden erwähnt werden: Ammoniak, Methylamin, Ethylamin, n-Propylamin, Isopropylamin, n-Butylamin, Isobutylamin, sek.-Butylamin, tert.-Butylamin, 2-Propenylamin, 2-Methylethenylamin, 2-Butenylamin, 3-Butenylamin, 1-Methyl-2-propenylamin, 2-Methyl-2-propenylamin, Propargylamin, 2-Butinylamin, 3-Butinylamin und 1-Methyl-2-propinylamin, Dimethylamin, Diethylamin, Di-n-propylamin, Di-n-butylamin, N-Methyl-ethylamin, N-Ethyl-n-propylamin, N-Methyl-allylamin und N-Methylpropargylamin.

Unter den Alkoholen, welche als Nucleophile eingesetzt werden können, sollen die folgenden erwähnt werden: Methanol, Ethanol, n-Propanol, i-Propanol, n-Butanol, i-Butanol, sek.-Butanol, tert.-Butanol, 2-Propenol, 2-Methylethenol, 2-Butenol, 3-Butenol, 1-Methyl-2-propenol, 2-Methyl-2-propenol, Propinol, 2-Butinol, 3-Butinol und 1-Methyl-2-propinol.

Unter den Thiolen, welche als Nucleophile eingesetzt werden können, sollen die folgenden erwähnt werden: Methanthiol, Ethanthiol, n-Propanthiol, i-Propanthiol, n-Butanthiol, i-Butanthiol, sek.-Butanthiol, tert.-Butanthiol, 2-Butenthiol, 2-Methylethenthiol, 2-Butenthiol, 3-Butenthiol, 1-Methyl-2-propenthiol, 2-Methyl-2-propenthiol, Propinthiol, 2-Butinthiol, 3-Butinthiol und 1-Methyl-2-propinthiol.

Die 4-Halogen-pyrimidine IIIb können in einem aprotisch polaren Lösungsmittel mit den Aminen XVI bei einer Temperatur von -80 bis +80 °C, vorteilhaft -30 bis +20 °C umgesetzt werden, wobei man das Amin XVI entweder in einem Überschuß einsetzt oder eine organische Hilfsbase verwendet.

Für die Umsetzung des 4-Halogen-pyrimidins IIIb mit dem Amin XVI sind folgende Lösungsmittel geeignet:

Ether wie Methyl-tert.-butylether, Diethylether, Ethylpropylether, n-Butylethylether, Di-n-butylether, Diisobutylether, Diisoamylether, Diisopropylether, Cyclohexylmethylether, Tetrahydrofuran, 1,2-Dimethoxyethan, Diethylenglykoldimethylether und Anisol, Ester wie Ethylacetat, n-Butylacetat und Isobutylacetat sowie chlorierte Kohlenwasserstoffe wie Methylenchlorid, 1,1,2,2-Tetrachlorethan, 1,1-Dichlorethylen, 1,2-Dichlorethan, Chlorbenzol, 1,2-Dichlorbenzol und 1-Chlornaphthalin und Gemische dieser Lösungsmittel.

Zweckmäßig verwendet man das Lösungsmittel in einer Menge von 100 bis 2000 Gew.%, vorzugsweise 400 bis 1200 Gew.%, bezogen auf den Ausgangsstoff IIIb.

Vorteilhaft gibt man 1,8 bis 2,5, insbesondere 1,95 bis 2,2 mol-Equivalente des Amins XVI, bezogen auf den Ausgangsstoff IIIb innerhalb 0,5 bis 2 Stunden zu einer Mischung von Ausgangsstoff IIIb in einem der vorgenannten Lösungsmittel bei (-80) bis 80 °C, vorzugsweise -30 bis 25 °C, rührt bis zur Vervollständigung der Reaktion (ca. 3 Stunden) nach und läßt dann zur Aufarbeitung auf 25 °C erwärmen.

Setzt man nur ungefähr stöchiometrische Mengen des Amins XVI ein, so setzt man zweckmäßig 0,9 bis 1,1 Equivalente einer organischen Hilfsbase, bezogen auf den Ausgangsstoff IIIb, zu. Als Hilfsbase eignen sich organische Basen wie Trimethylamin, Triethylamin, N-Ethyl-diisopropylamin, Triisopropylamin, N,N-Dimethylanilin, N,N-Dimethylcyclohexylamin, N-Methylpyrrolidin, Pyridin, Chinolin, $\alpha$-,$\beta$-, $\gamma$-Picolin, 2,4- und 2,6-Lutidin und Triethylendiamin.

Wird die Umsetzung mit Alkoholen oder Thiolen durchgeführt, kann man analog der für Amine beschriebenen Reaktionsweise verfahren. Vorteilhaft gibt man das Nucleophil in einer Menge von 0,9 bis 1,3 mol-Equivalenten, bezogen auf den Ausgangsstoff IIIb innerhalb 0,5 bis 2 Stunden zusammen mit einer der vorgenannten Hilfsbasen zu einer Mischung von Ausgangsstoff IIIb in einem der vorgenannten Lösungsmittel bei -30 bis 20 °C, rührt bis zur Vervollständigung der Reaktion (ca. 3 Stunden) nach und läßt dann zur Aufarbeitung auf 25 °C erwärmen.

Neben den genannten sind als Lösungsmittel auch Ketone; z. B. Aceton, Methylethylketon; dipolare aprotische Lösungsmittel, z. B. Acetonitril, Dimethylformamid, Dimethylacetamid, Dimethylsulfoxid, N-Methylpyrrolidon, 1,3-Dimethylimidazolin-2-on; Aromaten, z. B. Benzol, Toluol, Xylol oder entsprechende Gemische geeignet. Vorteilhaft kann man im Fall des Einsatzes von Alkoholen als Nucleophile diese direkt als Lösungsmittel verwenden. Besonders bevorzugt sind Salze von Alkoholen oder Thiolen, die den Einsatz einer organischen Hilfsbase entbehrlich machen. Sie werden auf bekannte Weise unter Verwendung von Alkali- oder Erdalkalimetallen oder Metallhydriden, z. B. NaH, KH, $CaH_2$ oder LiH hergestellt.

Die Reaktion kann drucklos oder unter Druck, kontinuierlich oder diskontinuierlich durchgeführt werden.

Zur Aufarbeitung extrahiert man das Umsetzungsgemisch mit Wasser zur Entfernung der Salze, trocknet und reinigt die organische Phase, z.B. durch Chromatographie. Die Umsetzungsprodukte sind jedoch meist genügend rein, so daß man nur von dem ausgefallenen Salz abzufiltrieren und die organische Phase einzuengen braucht.

Bevorzugte Zwischenprodukte der Formel IIIa sind beispielsweise:
2-Amino-4-methoxy-6-trifluormethoxy-pyrimidin,
2-Amino-4-chlordifluormethoxy-6-methoxy-pyrimidin,
2-Amino-4-ethoxy-6-trifluormethoxy-pyrimidin,
2-Amino-4-chlordifluormethoxy-6-ethoxy-pyrimidin,
2-Amino-4-allyloxy-6-trifluormethoxy-pyrimidin,
2-Amino-4-allyloxy-6-chlordifluormethoxy-pyrimidin,
2-Amino-4-methylthio-6-trifluormethoxy-pyrimidin,
2-Amino-4-chlordifluormethoxy-6-methylthio-pyrimidin,
2-Amino-4-ethylthio-6-trifluormethoxy-pyrimidin,
2-Amino-4-chlordifluormethoxy-6-ethylthio-pyrimidin,
2-Amino-4-methylamino-6-trifluormethoxy-pyrimidin,
2-Amino-4-chlordifluormethoxy-6-methylamino-pyrimidin,
2-Amino-4-ethylamino-6-trifluormethoxy-pyrimidin,
2-Amino-4-chlordifluormethoxy-6-ethylamino-pyrimidin,
2-Amino-4-dimethylamino-6-trifluormethoxy-pyrimidin,
2-Amino-4-chlordifluormethoxy-6-dimethylamino-pyrimidin,
4-Methoxy-2-methylamino-6-trifluormethoxy-pyrimidin,
4-Chlordifluormethoxy-6-methoxy-2-methylamino-pyrimidin,
4-Ethoxy-2-methylamino-6-trifluormethoxy-pyrimidin,
4-Chlordifluormethoxy-6-ethoxy-2-methylamino-pyrimidin,
2,4-Bis-methylamino-6-trifluormethoxy-pyrimidin,
4-Chlordifluormethoxy-2,6-bis-methylamino-pyrimidin,
4-Ethylamino-2-methylamino-6-trifluormethoxy-pyrimidin,
4-Chlordifluormethoxy-6-ethylamino-2-methylamino-pyrimidin,
4-Dimethylamino-2-methylamino-6-trifluormethoxy-pyrimidin,
4-Chlordifluormethoxy-6-dimethylamino-2-methylamino-pyrimidin

Ausführungsform C:

Man setzt ein Sulfonamid der Formel V in an sich bekannter Weise (EP-A-141 777) in einem inerten organischen Lösungsmittel mit ungefähr der stöchiometrischen Menge eines Phenylcarbamats VI bei einer Temperatur von 0 bis 120 °C, vorzugsweise 20 bis 100 °C um. Die Reaktion kann drucklos oder unter Druck (bis 50 bar), vorzugsweise bei 1 bis 5 bar, kontinuierlich oder diskontinuierlich durchgeführt werden.

Geeignete Lösungsmittel sind neben den in der oben zitierten Literatur aufgeführten, z.B. Nitrokohlenwasserstoffe wie Nitroethan und Nitrobenzol, Nitrile wie Acetonitril und Benzonitril, Ester wie Essigsäureethylester, Amide wie Dimethylformamid und/oder Ketone wie Aceton. Bevorzugt wird die Umsetzung in Essigsäureethylester als Lösungsmittel und mit Pyridin oder einem der vorstehend genannten tertiären Amin als Base.

Die Sulfonamide der Formel V lassen sich durch Reaktion der entsprechenden Sulfonsäurechloride mit Ammoniak gewinnen (M. Quaedvlieg in Houben-Weyl, "Methoden der organischen Chemie", Georg Thieme Verlag, Stuttgart, Bd. 9 (1955) 398-400, F. Muth, ibid., 605ff.). Man kann jedoch auch in einer nukleophilen Substitution ein o-Halogenbenzolsulfonamid z.B. mit einem Alkohol oder Thiol umsetzen und beispielsweise den entstandenen Thioether zum Sulfoxid oder Sulfon oxydieren (siehe Verfahrensbeispiele).

Die entsprechenden Sulfonsäurechloride zur Herstellung der Sulfonamide der Formel V erhält man allgemein durch Meerwein-Reaktion (Diazotierung geeigneter Amine und Kupfersalz-katalysierte Sulfochlorierung mit Schwefeldioxid: F. Muth in Houben-Weyl, "Methoden der organischen Chemie", Georg Thieme Verlag, Stuttgart, Bd. 9 (1955) 579, S. Pawlenko in Houben-Weyl, "Methoden der organischen Chemie", Georg Thieme Verlag, Stuttgart, Bd. E 11/2 (1985) 1069), aus den entsprechenden Sulfonsäuren (F. Muth in Houben-Weyl, "Methoden der organischen Chemie", Georg Thieme Verlag, Stuttgart, Bd. 9 (1955) 564), durch Chlorsulfonierung geeigneter aromatischer Vorstufen (F. Muth, ibid., S. 572) oder durch oxydative Chlorierung niederwertiger Schwefelvorstufen (Mercaptane, Diaryldisulfide, S-Benzylmercaptane) (F. Muth, ibid., S. 580, S. Pawlenko, loc. cit., S. 1073). Ortho-cyanosubstituierte Benzolsulfonsäurechloride lassen sich oft vorteilhaft durch Ringöffnung entsprechender Saccharine mit Phosphorpentachlorid erhalten (J. Chem. Soc. 89 (1906) 352).

Die als Ausgangsstoffe benötigten Sulfonylisocyanate der Formel II lassen sich in an sich bekannter Weise aus den entsprechenden Sulfonamiden durch Phosgenierung (Houben-Weyl 11/2 (1985) 1106, US 4 379 769) oder durch Umsetzung der Sulfonamide mit Chlorsulfonylisocyanat (DE-OS 3 132 944) gewinnen.

Die Sulfonylcarbamate der Formel IV wurden nach oder in Analogie zu an sich bekannten Reaktionen (z.B. EP-A 120 814) hergestellt. Man kann aber auch die Sulfonylisocyanate der Formel I in glatter Reaktion in einem inerten Lösungsmittel wie Ether oder Dichlormethan mit Phenol in die Carbamate der Formel IV überführen.

Carbamate der Formel IV sind nach oder in Analogie zu bekannten Umsetzungen (z.B. EP-A 101 670) zugänglich, sie lassen sich aber auch aus entsprechenden Isocyanaten durch Umsetzung mit Phenol herstellen.

Die Isocyanate erhält man aus den Amiden der Formel III durch Behandlung mit Oxalylchlorid oder Phosgen (in Analogie nach Angew. Chem. 83 (1971) 407, EP-A 388 873).

Sulfonylharnstoffe der Formel I, die die unter Anspruch 8 spezifizierte Bedeutung haben, lassen sich auch durch Umsetzung von Sulfonierungs- oder Acylierungsreagenzien VIb,d mit 2-Amino-, 2-Alkylamino- oder 2-Hydroxybenzolsulfonylharnstoffen VIa bzw. VIc herstellen.

2-Aminobenzolsulfonylharnstoffe der Formel VIa gewinnt man durch katalytische Hydrierung der entsprechend substituierten 2-Nitrobenzolsulfonylharnstoffe in einem inerten organischen Lösungsmittel, wie bspw. Methanol, Ethanol, Dioxan oder Essigsäureethylester, wobei als Katalysatoren Metall oder Metalloxide auf Trägern wie Pd/Aktivkohle, Raney-Nickel oder $PtO_2$ verwendet werden können (Autorenkollektiv in "Organikum", VEB Deutscher Verlag der Wissenschaften, Berlin (1955), 645-649).

2-Alkylaminobenzolsulfonylharnstoffe der Formel VIa stellt man aus dem entsprechend substituierten 2-Aminobenzosulfonylharnstoff durch Alkylierung mit geeigneten Alkylhalogeniden, Dialkylsulfaten oder Alkyltosylaten her.

2-Hydroxybenzolsulfonylharnstoffe der Formel VIc stellt man bspw. durch katalytische Hydrierung geeignet substituierter 2-Benzyloxybenzolsulfonylharnstoffe dar, wobei die bereits oben erwähnten Lösungsmittel und Katalysatoren Verwendung finden.

Man setzt einen Sulfonylharnstoff der Formel IVa,c in an sich bekannter Weise (J. March in "Abvanced Organic Chemistry", J. Wiley & Sons, New York (1985), S. 370-371, 346-351 und dort zit. Literatur) in einem inerten organischen Lösungsmittel mit ungefähr der stöchiometrischen Menge des Acylierungs- bzw. Sulfonierungsreagenzes VIb,d in Gegenwart einer Hilfsbase VIe bei einer Temperatur von 0 bis 120 °C, vorzugsweise 0 bis 100 °C um. Die Reaktion kann drucklos oder unter Druck (bis 50 bar), vorzugsweise bei

1 bar, kontinuierlich oder diskontinuierlich durchgeführt werden. Geeignete Lösungsmittel sind in der oben genannten Literatur aufgeführt, z.B. Acetonitril, Tetrahydrofuran, Essigsäureethylester, Dimethylformamid, N-Methyl-2-pyrrolidon oder Aceton. Geeignete Hilfsbasen VIe sind die für die Verfahrensvariante B aufgeführten tertiären Amine oder Alkalimetallcarbonate. Bevorzugt wird die Reaktion in Acetonitril, Tetrahydrofuran oder Dimethylformamid in Gegenwart von Pyridin oder Kaliumcarbonat durchgeführt.

Im Hinblick auf die biologische Wirksamkeit sind Verbindungen der Formel I oder deren Salze bevorzugt, in denen die Substituenten folgende Bedeutung haben:

$R^1$ Wasserstoff und Methyl,

$R^2$ Fluor, Chlor, Brom und Trifluormethyl, (m = 0), ferner Methyl, Ethyl, n-Propyl und Isopropyl (m = 1),

$R^3$ Wasserstoff, Fluor, Chlor, Brom, Methyl, Methoxy, Trifluormethyl und Trifluormethoxy,

X Sauerstoff, Schwefel und eine Aminogruppe $-NR^4$, wobei

$R^4$ für Wasserstoff, Methyl und Ethyl steht,

A $NO_2$, $NH_2$, OH, CN, SCN, eine Ethergruppe wie $OCH_3$ oder $OC_2H_5$, wobei die Methylgruppe noch 1 bis 3, die Ethylgruppe noch 1 bis 4 Halogenatome wie insbesondere Fluor oder Chlor oder beide Reste eine Methoxygruppe tragen können, eine Sulfidgruppe, eine Sulfoxidgruppe, eine Sulfongruppe, eine Sulfonamidgruppe, z.B. $SO_2$N-di-$C_1$-$C_4$-alkylamino, ein Carbonat, eine Acyloxy- oder eine Acylgruppe, z.B. Acetyloxy oder Acetyl,

$R^5$ eine $C_1$-$C_3$-Alkylgruppe wie Methyl, Ethyl, n-Propyl und Isopropyl, eine Alkenylgruppe wie Allyl, Crotyl und But-1-en-3-yl ;
eine Alkinylgruppe wie Propargyl, But-1-in-3-yl und But-2-inyl;
Halogenalkyl wie 2-Chlorethyl, 2-Chlor-n-propyl, 3-Chlor-n-propyl, 1-Chlorbut-2-yl, 2-Chlor-isobutyl, 4-Chlor-n-butyl, Chlor-tert.-butyl, 3-Chlor-prop-2-yl, 2,2,2-Trifluorethyl und Trifluormethyl ;
Alkoxyalkyl wie 2-Methoxyethyl, 3-Ethoxyethyl, 3-Methoxy-n-propyl, 2-Methoxy-n-propyl, 3-Methoxy-n-butyl, 1-Methoxy-but-2-yl, Methoxy-tert.-butyl, 2-Methoxy-n-butyl und 4-Methoxy-n-butyl;
Alkoxyalkoxyalkyl wie 2-Methoxy-ethoxy-methyl, 2-(Ethoxy)-ethoxy-methyl, 2-(Propoxy)-ethoxy-methyl, 2-Methoxy-ethoxy-ethyl, 2-(Ethoxy)-ethoxy-ethyl und 2-(Methoxy-methoxy)-ethyl ;
Halogenalkoxyalkyl wie 2-($\beta$-Chlorethoxy)-ethyl, 3-($\beta$-Chlorethoxy)-n-propyl und 3-($\gamma$-Chlor-n-propoxy)-n-propyl;
Cycloalkyl wie Cyclopropyl, Cyclopentyl und Cyclohexyl,

$R^6$ Wasserstoff;
Alkoxy wie Methoxy oder Ethoxy;
Alkyl wie Methyl, Ethyl, n-Propyl, Isopropyl und n-Butyl
oder gemeinsam mit $R^7$ Tetramethylen, Pentamethylen, Hexamethylen, Ethylenoxyethylen und Ethylen-N-methyliminoethylen,

$R^7$ Alkyl wie Methyl, Ethyl, n-Propyl, Isopropyl
Halogenalkyl wie oben für $R^5$ genannt, Difluormethyl, Trifluormethyl, Chlordifluormethyl, 1,1,2,2-Tetrafluorethyl, 2,2,2-Trifluorethyl, 2-Chlor-1,1,2-trifluorethyl;
Alkoxyalkyl wie oben für $R^5$ genannt;
Cycloalkyl wie Cyclopropyl, Cyclobutyl, Cyclopentyl;

$R^8$ Wasserstoff oder Halogen wie Fluor oder Chlor, bevorzugt Wasserstoff;

m,n o,p,q
0 oder 1, oder o zusätzlich 2, wobei im Falle von p = 0 q ebenfalls für 0 steht.

Besonders bevorzugt sind Sulfonylharnstoffe der Formel I, in der die Substituenten folgende Bedeutung haben:

$R^1$ Wasserstoff oder Methyl;

$R^2$ Halogen oder Trifluormethyl, wenn m für 0 steht und Methyl, wenn m 1 bedeutet;

X O oder NH;

$R^3$ Wasserstoff, Halogen, Methyl oder Methoxy;

A $NO_2$, $N[CH_3]SO_2CH_3$, eine Gruppe $SO_2R^5$, worin $R^5$ $C_1$-$C_4$-Alkyl bedeutet, eine Gruppe $SO_2NR^6R^7$, worin $R^6$ und $R^7$ Methyl bedeuten, eine $OR^7$-Gruppe, worin $R^7$ $C_1$-$C_2$-Alkyl, das ein bis drei bzw. vier Halogenatome oder eine Methoxygruppe tragen kann, bedeutet;

$R^8$ Wasserstoff;

sowie deren umweltverträgliche Salze.

Die Salze der Verbindungen I sind in an sich bekannter Weise zugänglich (EP-A-304 282, US-A 4,599,412). Man erhält sie durch Deprotonierung der entsprechenden Sulfonylharnstoffe I in Wasser oder einem inerten organischen Lösungsmittel bei Temperaturen von -80 °C bis 120 °C, vorzugsweise 0 °C bis 60 °C in Gegenwart einer Base.

Geeignete Basen sind beispielsweise Alkali- oder Erdalkalimetallhydroxide, -hydride, -oxide oder -alkoholate wie Natrium-, Kalium- und Lithiumhydroxid, Natriummethanolat, -ethanolat und -tert.-butanolat, Natrium- und Calziumhydrid und Calziumoxid. Durch Kationenaustausch lassen sich daraus Salze mit anderen Gegenionen, wie Ammonium, Tetraalkylammonium, Benzyltrialkylammonium, Phosphonium, Sulfonium o.ä. herstellen.

Als Lösungsmittel kommen beispielsweise neben Wasser auch Alkohole wie Methanol, Ethanol und tert.-Butanol, Ether wie Tetrahydrofuran und Dioxan, Acetonitril, Dimethylformamid, Ketone wie Aceton und Methylethylketon und auch halogenierte Kohlenwasserstoffe in Betracht.

Die Deprotonierung kann bei Normaldruck oder bei Drucken bis 50 bar, vorzugsweise bei Normaldruck bis 5 bar Überdruck durchgeführt werden.

Als Salze der Verbindungen der Formel I kommen landwirtschaftlich brauchbare Salze, beispielsweise Alkalimetallsalze wie das Kalium-oder Natriumsalz, Erdalkalimetallsalze, wie das Calcium-, Magnesium- oder Bariumsalz; Mangan-, Kupfer-, Zink- oder Eisensalze sowie Ammonium, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Die erfindungsgemäßen herbiziden und wachstumsregulierenden Verbindungen I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier-oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.003 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.005 werden in einer Mischung gelöst, die aus 80 Gewichtsteilen Xylol, 10 Gewichtsteilen des Anlagerungsproduktes von 8 bis 10 mol Ethylenoxid an 1 mol Ölsäure-N-monoethanolamid, 5 Gewichtsteilen Calciumsalz der Dodecylbenzolsulfonsäure und 5 Gewichtsteilen des Anlagerungsproduktes von 40 mol Ethylenoxid an 1 mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 2.005 werden in einer Mischung gelöst, die aus 40 Gewichtsteilen Cyclohexanon, 30 Gewichtsteilen Isobutanol, 20 Gewichtsteilen des Anlagerungsproduktes von 7 mol Ethylenoxid an 1 mol Isooctylphenol und 10 Gewichtsteilen des Anlagerungsproduktes von 40 mol Ethylenoxid an 1 mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 5.001 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 mol Ethylenoxid an 1 mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 5.003 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 6.001 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 9.001 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 9.011 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff bei Anwendung als Herbizide betragen je nach Bekämpfungsziel, Jahreszeit, Zielpflanzen und Wachstumsstadium 0,001 bis 5, vorzugsweise 0,01 bis 2 kg/ha aktive Substanz (a.S.).

Aus der Reihe der verschiedenartigen Anwendungsmöglichkeiten der erfindungsgemäßen Verbindungen als Pflanzenwachstumsregulatoren der Formel I im Pflanzenanbau, in der Landwirtschaft und im Gartenbau, werden eingige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen.

Die Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen eingesetzt werden.

Kulturenliste:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rübsen |
| Camellia sinensis | Teestrauch |
| Carthamus tinctorius | Saflor-Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |

| Botanischer Name | Deutscher Name |
|---|---|
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffel |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Micotiana tabacum (N. rustica) | Takak |
| Olea europaea | Ölbaum |
| Oryza sativa | Reis |
| Phaseolus lunatus | Mondbohne |
| Phaseolus vulgaris | Buschbohne |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (S. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazin, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Beispielen wiedergegeben Vorschriften wurden unter entsprechender Abwandlung der Ausgangsverbindungen zur Gewinnung weiterer Verbindungen der Formel I benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellen und untersuchten Verbindungen eine gleichartige Wirkung erwarten.

I Herstellung der Vorprodukte

Beispiel I.1

2-Chlor-4-trichlormethoxy-6-trichlormethylpyrimidin

a) 2-Chlor-4-methoxy-6-trichlormethylpyrimidin

293,1 g (1,692 mol) 30 %ige Natriummethylatlösung wurden innerhalb 1 1/2 Stunden bei 0 bis 5°C unter Rühren zu einer Lösung von 434 g (1,692 mol) 2,6-Dichlor-4-trichlormethylpyrimidin in 1 l 1,2-Dichlorethan gegeben. Es wurde 1 Stunde bei 0 bis 5°C und 12 Stunden bei 25°C gerührt. Das Reaktionsgemisch wurde mit Wasser und mit gesättigter Kochsalzlösung extrahiert. Nach dem Trocknen über Magnesiumsulfat und Einengen erhielt man 423 g (95 % d. Th.) der Titelverbindung als fast farbloses Öl vom $n_D^{23}$ = 1.5552. $^1$H-NMR ($CDCl_3$) (ppm) $OCH_3$ (s/3H) 4,1; CH (s/1H) 7,25.

b) 2-Chlor-4-trichlormethoxy-6-trichlormethylpyrimidin

In eine Mischung von 210 g (0,802 mol) a) und 260 mg (0,0016 mol) $\alpha,\alpha'$-Azoisobutyronitril wurden unter UV-Bestrahlung und gaschromatographischer Kontrolle des Reaktionsverlaufs bei einer Temperatur von zunächst 110°C Chlor eingeleitet, wobei sich auch nach Entfernen der Heizbades eine Reaktionstemperatur von 140°C einstellte. Nach dem Abklingen der Reaktion wurden während 5 1/2 Stunden bei 120°C insgesamt 341 g (4,8 mol) Chlor eingeleitet. Zu dem erkaltenden Reaktionsgemisch rührte man ab 40°C 70 ml n-Pentan zur Ausfällung hinzu. Der Niederschlag wurde abgesaugt, mit Petrolether gewaschen und getrocknet, wobei man 163 g (55 % d. Th.) der Titelverbindung vom Fp. 67-69°C erhielt.

Das Filtrat (113,8 g) bestand nach dem Gaschromatogramm zu 83 % aus der Titelverbindung, 4 % 2-Chlor-4-dichlormethoxy-6-trichlormethylpyrimidin und 9 % 2,4-Dichlor-6-trichlormethylpyrimidin. Die Gesamtausbeute der Titelverbindung betrug 87,6 % d. Th.

Beispiel I.2

2,4-Difluor-6-trichlormethoxy-pyrimidin

a) 2,4-Difluor-6-methoxypyrimidin

(Herstellung nach dem Verfahren der älteren deutschen Patentanmeldung P 39 00 471 (O.Z. 0050/40474)

Zu einer Mischung aus 250 g (1,865 mol) 2,4,6-Trifluorpyrimidin in 1,4 l Methanol wurden bei -20°C innerhalb von 45 Minuten 335,8 g (1,865 mol) 30 %iges Natriummethylat (in Methanol) gegeben und weitere 30 Minuten bei dieser Temperatur gerührt. Anschließend ließ man auf 25°C erwärmen und engte

die Reaktionsmischung auf ca. 1/5 ihres Volumens ein.

Das so erhaltene Gemisch wurde zwischen Diethylether und Wasser verteilt, wonach die organische Phase über Magnesiumsulfat getrocknet und eingeengt wurde. Nach Destillation (1,1 m Kolonne, 3 mm V-Füllkörper) erhielt man 141,6 g (52 % d. Th.) der Titelverbindung von Kp,: 144-145 °C.

Aus dem Destillationsrückstand erhielt man durch Destillation über einen Normag-Aufsatz 114,4 g (42 % d. Th.) an 4,6-Difluor-2-methoxy-pyrimidin vom Kp.: 157-161 °C.

b) 2,4-Difluor-6-trichlormethoxypyrimidin

210 g (2,96 mol) Chlor wurden unter UV-Bestrahlung und gaschromatographischer Kontrolle des Reaktionsverlaufs während 2 1/2 Stunden unter Rühren bei 130 °C in 123 g (0,843 mol) 2,4-Difluor-6-methoxy-pyrimidin eingeleitet. Das Reaktionsgemisch wurde über eine 10-cm-Vigreux-Kolonne im Vakuum destilliert, wobei man 190,2 g (90,5 % d. Th.) der Titelverbindung vom Sdp. 40-43 °C/0,2 mbar erhielt.

Beispiel I.3

2,4-Dichlor-6-trichlormethoxy-pyrimidin

Unter Rühren, UV-Bestrahlung und gaschromatographischer Kontrolle des Reaktionsverlaufs wurden 303 g (4,27 mol) Chlor während 1/2 Stunde bei 80 °C, 1 Stunde bei 100 °C, 3 Stunden bei 120 °C und 3 Stunden bei 150 °C in eine Mischung von 209 g (1,168 mol) 2,6-Dichlor-4-methoxy-pyrimidin und 2 g (0,012 mol) $\alpha,\alpha'$-Azoisobutyronitril eingeleitet. Anschließend wurde das Reaktionsgemisch im Vakuum über eine 50-cm-Kolonne mit 4 mm V2-A-Raschigringen destilliert. Man erhielt 241,3 g (73 % d. Th.) der Titelverbindung vom Sdp. 87-88 °C/0,4 mbar; Fp. 55-56 °C.

Beispiel I.4

2,4-Difluor-6-trifluormethoxy-pyrimidin

49,9 g (0,2 mol) 2,4-Difluor-6-trichlormethoxy-pyrimidin wurde bei 100 °C innerhalb 15 Minuten unter Rühren zu einer Mischung von 39,3 g (0,22 mol) Antimontrifluorid und 9,38 g (0,031 mol) Antimonpentachlorid gegeben.

Innerhalb 25 Minuten wurde die Badtemperatur von 100 auf 150 °C erhöht und 30 Minuten nachgerührt, wobei sich zwischen 120 bis 125 °C Rückfluß einstellte. Durch anschließende Destillation erhielt man 37,1 g (92,7 % d. Th.) der Titelverbindung vom Sdp. 125-127 °C und $n_D^{23}$ = 1.3787.

Beispiel I.5

6-Chlordifluormethoxy-2,4-difluor-pyrimidin

93 g (0,373 mol) 2,4-Difluor-6-trichlormethoxy-pyrimidin wurden bei 100 °C innerhalb 10 Minuten unter Rühren zu einer Mischung von 44,5 g (0,249 mol) Antimontrifluorid und 0,94 g (0,0031 mol) Antimonpentachlorid gegeben. Innerhalb 25 Minuten wurde die Badtemperatur von 100 auf 175 °C erhöht, wobei sich bei 145 °C Rückfluß einstellte. Nach 1 1/2 Stunden Rühren wurde das Reaktionsprodukt bei 146-150 °C abdestilliert. Das Destillat wurde in 200 ml Methylenchlorid gelöst, 2x mit 6 n Salzsäure extrahiert und über Magnesiumsulfat getrocknet. Nach dem Einengen im Vakuum erhielt man als Rückstand die Titelverbindung vom $n_D^{23}$ = 1.4142 in einer Ausbeute von 63,7 g = 78,8 % d.Th.

Beispiel I.6

2-Fluor-4-trifluormethoxy-6-trifluormethyl-pyrimidin

80 g (0,219 mol) 2-Chlor-4-trichlormethyl-6-trichlormethoxypyrimidin wurden innerhalb 5 Minuten unter Rühren bei 100 °C zu einer Mischung von 93,9 g (0,525 mol) Antimontrifluorid und 18,7 g (0,0627 mol) Antimonpentachlorid gegeben. Innerhalb 10 Minuten wurde die Badtemperatur auf 140 °C erhöht und 1 Stunde nachgerührt, wobei sich starker Rückfluß einstellte. Das Reaktionsprodukt wurde bei 135-140 °C, gegen Schluß bei 95 °C/50 mbar, überdestilliert. Das Destillat wurde in Methylenchlorid aufgenommen, mit 6 n Salzsäure extrahiert und über Magnesiumsulfat getrocknet. Nach dem Einengen im Vakuum erhielt man

die Titelverbindung in einer Ausbeute von 35,9 g (65,5 % d. Th.).

Beispiel I.7

2,4-Dichlor-6-trifluormethoxy-pyrimidin

115 g (0,407 mol) 2,4-Dichlor-6-trichlormethoxy-pyrimidin wurden innerhalb 5 Minuten unter Rühren bei 100 °C zu einer Mischung von 80 g (0,447 mol) Antimontrifluorid und 18,77 g (0,0627 mol) Antimonpentachlorid gegeben, wobei sich die Reaktionstemperatur bis auf 140 °C erhöhte. Es wurde noch 45 Minuten bei 150 °C gerührt. Zur Destillation wurde ein Druck von 210 mbar eingestellt, wobei die Titelverbindung bei 128 °C überging; letzte flüchtige Bestandteile wurden bei 110 °C/22 mbar übergetrieben. Das Destillat wurde in Methylenchlorid gelöst, 3x mit 6 n Salzsäure extrahiert und über Magnesiumsulfat getrocknet. Nach dem Einengen im Vakuum erhielt man die Titelverbindung in einer Ausbeute von 80 g (84,4 % d. Th.) als farbloses Öl vom $n_D^{25}$ = 1,4604.

Beispiel I.8

2-Amino-4-chlordifluormethoxy-6-fluor-pyrimidin

9,8 g (0,578 mol) gasförmiges Ammoniak wurden innerhalb einer Stunde bei -75 bis -70 °C unter Rühren in eine Mischung von 62,5 g (0,289 mol) 2,4-Difluor-6-chlordifluormethoxy-pyrimidin in 300 ml Tetrahydrofuran eingegast. Es wurde eine Stunde bei -70 °C gerührt und dann auf Raumtemperatur erwärmt. Der ausgefallene Niederschlag wurde abgesaugt, zwischen Essigester und Wasser verteilt und die organische Phase über Magnesiumsulfat getrocknet. Das Reaktionsfiltrat wurde eingeengt, in der obigen Essigesterphase gelöst, über Kieselgel mit Petrolether:Ether = 5:1 chromatographiert und eingeengt. Man erhielt 46,5 g (75,3 % d. Th.) der Titelverbindung als farblose Kristalle vom Fp. 77-80 °C.

Beispiel I.9

2-Amino-4-fluor-6-trifluormethoxy-pyrimidin

8,7 g (0,51 mol) gasförmiges Ammoniak wurden innerhalb 1 Stunde bei -75 bis 70 °C unter Rühren in eine Mischung von 51 g (0,255 mol) 2,4-Difluor-6-trifluormethoxy-pyrimidin in 200 ml Diethylether eingegast. Es wurde noch 1 1/2 Stunden bei -70 °C und 1 Stunde bei Raumtemperatur gerührt. Das Reaktionsgemisch wurde im Vakuum eingeengt, in Methylenchlorid aufgenommen und mit Wasser extrahiert. Nach dem Trocknen der organischen Phase, Einengen und Chromatographieren über Kieselgel mit Petrolether:Ether = 8:1 erhielt man 38,1 g (75,6 % d. Th.) der Titelverbindung als farblose Kristalle vom Fp. 86-89 °C.

Beispiel I.10

2-Amino-4-chlor-6-trifluormethoxy-pyrimidin

4,3 g (0,25 mol) gasförmiges Ammoniak wurden innerhalb 45 Minuten unter Rühren bei -50 bis -45 °C in eine Mischung von 23,3 g (0,1 mol) 2,4-Dichlor-6-trifluormethoxy-pyrimidin in 150 ml Methyl-tert.-butylether eingeleitet. Es wurde 30 Minuten bei -50 °C, 1 Stunde bei -30 °C und 1 Stunde bei 25 °C gerührt. Der ausgefallene Niederschlag wurde abgesaugt, mit Wasser gewaschen und getrocknet, wobei man 5,4 g (33,1 % d. Th.) 4-Amino-2,4-dichlorpyrimidin vom Fp. 270-272 als Nebenprodukt erhielt. Das Filtrat wurde mit Wasser gewaschen, getrocknet, teilweise im Vakuum eingeengt und mit Petrolether:Ether = 5:1 frakioniert chromatographiert, wobei man in den ersten Fraktionen 3 g (12,8 % d. Th.) Ausgangsmaterial als farbloses Öl und im Nachlauf 9 g (42 % d. Th.) der Titelverbindung als farblose Kristalle vom Fp. 55-56 °C erhielt. Der Umsatz betrug 48,3 %.

Beispiel I.11

4-Chlordifluormethoxy-6-fluor-2-methylamino-pyrimidin

20,3 g (0,0938 mol) 4-Chlordifluormethoxy-2,6-difluor-pyrimidin wurden in 150 ml Tetrahydrofuran vorgelegt und bei -70 bis -60 °C innerhalb 30 Minuten unter Rühren mit 5,8 g (0,188 mol) gasförmigem

Methylamin versetzt. Es wurde jeweils 1 Stunde bei -70 °C, 0 °C und 25 °C gerührt. Nach dem Einengen des Reaktionsgemisches im Vakuum wurde der Rückstand mit Wasser verrührt, 2x mit Essigester extrahiert und der Extrakt über Magnesiumsulfat getrocknet. Es wurde zum Teil im Vakuum eingeengt und dann über Kieselgel mit Ether/Petrolether 1:5 fraktioniert chromatographiert. Die ersten Fraktionen enthielten die Titelverbindung vom Fp. 57-61 °C in einer Ausbeute von 12,5 g (58,5 %).

Beispiel I.12

2-Amino-4-trifluormethoxy-6-trifluormethyl-pyrimidin

4,7 g (0,278 mol) gasförmiges Ammoniak wurden unter Rühren bei -75 bis -70 °C innerhalb 1 Stunde in eine Mischung von 38,0 g (0,147 mol) 2-Fluor(chlor)-4-trifluormethoxy-6-trifluormethylpyrimidin in 150 ml Diethylether eingegast. Es wurde jeweils 2 Stunden bei -75 und nach dem Erwärmen bei 25 °C gerührt. Nach dem Absaugen von dem ausgefallenen Niederschlag wurde die organische Phase mit Wasser extrahiert, getrocknet und teilweise eingeengt. Nach dem Chromatographieren mit Methyl-tert.-butylether über Kieselgel erhielt man 20,4 g (56,1 % d. Th.) der Titelverbindung vom Fp. 47-49 °C.

II. Herstellung der Zwischenprodukte IIIa

Beispiel II.1

2-Amino-4-methoxy-6-trifluormethoxy-pyrimidin

2,7 g (0,015 mol) 30 %iges Natriummethylat wurden innerhalb 15 Minuten unter Rühren bei -5 bis 0 °C zu 2,95 g (0,015 mol) 2-Amino-4-fluor-6-trifluormethoxy-pyrimidin in 50 ml Methanol gegeben. Nach 1 Stunde Rühren bei 0 °C und Erwärmen auf 25 °C wurde das Reaktionsgemisch im Vakuum eingeengt, mit Wasser verrührt und mit Methylenchlorid extrahiert. Nach dem Trocknen und Einengen im Vakuum erhielt man 3,1 g (98 % d. Th.) der Titelverbindung mit $n_D^{25}$ = 1,4770.

Beispiel II.2

2-Amino-4-chlordifluormethoxy-6-methoxy-pyrimidin

26,1 g (0,145 mol) 30 %iges Natriummethylat wurden innerhalb 15 Minuten unter Rühren bei -10 bis 0 °C zu 31,0 g (0,145 mol) 2-Amino-4-chlordifluormethoxy-6-fluorpyrimidin in 300 ml Methanol gegeben. Es wurde 30 Minuten bei 0 °C und 1 Stunde bei 25 °C gerührt. Das Reaktionsgemisch wurde im Vakuum eingeengt und wie oben aufgearbeitet. Man erhielt 31,6 g (96,6 % d. Th.) der Titelverbindung als farbloses Öl mit $n_D^{22}$ = 1,5039.

Beispiel II.3

4-Chlordifluormethoxy-2-methylamino-6-methoxy-pyrimidin

4,7 g (0,026 mol) 30 %iges Natriummethylat wurden innerhalb 10 Minuten unter Rühren bei 0 °C zu 6,0 g (0,0263 mol) 4-Chlordifluormethoxy-6-fluor-2-methylamino-pyrimidin in 100 ml Methanol gegeben. Es wurde jeweils 1 Stunde bei 0 °C und bei 25 °C gerührt. Nach üblicher Aufarbeitung erhielt man 6,3 g (100 % d. Th.) der Titelverbindung vom Fp. 49-53 °C.

Beispiel II.4

4-Chlordifluormethoxy-6-dimethylamino-2-methylamino-pyrimidin

1,9 g (0,0417 mol) gasförmiges Dimethylamin wurden innerhalb 10 Minuten unter Rühren bei 0 °C in eine Mischung von 8,9 g (0,0417 mol) 2-Amino-4-chlordifluormethoxy-6-fluor-pyrimidin in 100 ml Tetrahydrofuran eingeleitet. Es wurde 1 Stunde bei 0 °C und 2 Stunden bei 25 °C gerührt. Nach üblicher Aufarbeitung erhielt man 9,7 g (97,5 % d. Th.) der Titelverbindung vom Fp. 127-130 °C.

III. Herstellung der Vorprodukte II

2-(Ethylsulfonyl)benzolsulfonylisocyanat

a) 2-(Ethylthio)benzolsulfonamid

62 g (1,0 mol) Ethanthiol wurden bei 25 °C unter Rühren zu einer Mischung von 65,9 g (1,0 mol) 85 %igem Kaliumhydroxidpulver und 500 ml Dimethylformamid gegeben und 15 Minuten nachgerührt. Anschließend wurde bei 90 °C unter Rühren innerhalb 30 Minuten eine Lösung von 95,8 g (0,5 mol) 2-Chlorbenzolsulfonamid zugegeben und 8 Stunden bei 110 °C gerührt. Nach dem Abkühlen und Einengen im Vakuum wurde der Rückstand zwischen Methylenchlorid/Wasser verteilt und die organische Phase mit verdünnter Kochsalzlösung gewaschen. Nach dem Einengen im Vakuum erhielt man 88,4 g (81,5 % d.Th.) der Titelverbindung als halbkristalline Masse.

b) 2-(Ethylsulfonyl)benzolsulfonamid

81,6 g (1,2 mol) 50 %iges Wasserstoffsuperoxid wurden innerhalb 30 Minuten unter Rühren bei 60 °C zu einer Mischung von 88 g (ca. 0,4 mol) rohem 2-(Ethylthio)benzolsulfonamid in 200 ml Eisessig gegeben und über Nacht bei 25 °C nachgerührt. Nach weiteren 4 Stunden Rühren bei 60 °C wurde abgekühlt und auf 500 ml Eiswasser gegossen. Der ausgefallene Niederschlag wurde abgesaugt, mit Wasser gewaschen und im Vakuum bei 50 °C getrocknet, wobei man 72,3 g (72,5 % d.Th.) der Titelverbindung vom Fp 179-181 °C erhielt.

c) 2-(Ethylsulfonyl)benzolsulfonylisocyanat

102,8 g (0,865 mol) Thionylchlorid wurden innerhalb 30 Minuten unter Rühren bei 70 bis 80 °C zu 71,8 g (0,288 mol) 2-(Ethylsulfonyl)benzolsulfonamid in 500 ml 1,2-Dichlorethan gegeben und 2,5 Stunden unter Rückfluß gerührt. Nach dem Abkühlen auf 50 °C wurden 2 ml Pyridin zugegeben und während 5 Stunden mit Phosgen unter Rückfluß und Rühren begast. Nach dem Einengen erhielt man 84,1 g der Titelverbindung, die zur Aufbewahrung direkt in 1,2-Dichlorethan aufgenommen wurde.

2-(Methylsulfinyl)benzolsulfonylisocyanat

a) 2-(Methylsulfinyl)benzolsulfonamid

Zu einer Suspension von 26,5 g 2-(Methylthio)benzolsulfonamid (0,13 mol) (hergestellt in analoger Weise zum 2-(Ethylthio)-benzolsulfonamid) und 2,1 g $Na_2WO_4 \cdot 2H_2O$ in 88 ml Eisessig tropft man bei einer Temperatur zwischen 25 und 30 °C 14,8 g Wasserstoffperoxid (30 % in $H_2O$) (0,13 mol). Die Suspension klärt sich zu einer homogenen Lösung, aus der sich rasch ein Niederschlag abscheidet. Man rührt 45 min bei 25 °C, gießt den Ansatz auf 400 ml $H_2O$ und saugt den Niederschlag ab. Dieser wird mit Wasser gewaschen und im Wasserstrahlvakuum bei 40 °C getrocknet. Man erhält so 24,3 g (85 % d.Th.) der Titelverbindung. $^1$H-NMR-Spektrum (250 MHz, $CD_3SOCD_3$, int. TMS): 8,16 d (1H), 7,82-8,0 m (2H), 7,77 br (2H), 7,63-7,85 m (2H), 2,76 s (3H).

b) N-(n-Butylamino)carbonyl-2-methylsulfinylbenzolsulfonamid

Zu einer Suspension von 20,1 g 2-(Methylsulfinyl)benzolsulfonamid (0,09 mol) in 250 ml Acetonitril tropft man bei 25 °C 10,2 g n-Butylisocyanat (0,10 mol). Nach Zugabe von 13,9 g Kaliumcarbonat (0,10 mol) rührt man 4 h unter Rückfluß. Nach Abkühlen auf 0 °C gießt man auf 400 ml Eis/Wasser, stellt durch Zugabe von konz. Salzsäure einen pH-Wert von 1 ein und extrahiert 2x mit je 250 ml Methylenchlorid. Die organischen Extrakte werden mit Wasser neutral gewaschen und über $Na_2SO_4$ getrocknet. Nach Entfernen des Lösungsmittels erhält man 25,0 g der Titelverbindung (85 % d.Th.) als hellbraunes Öl.
$^1$H-NMR-Spektrum (250 MHz, $CDCl_3$, int. TMS): 8,28 d (1H), 7,89 t (1H), 7,73 t (1H), 6,03 t (1H), 3,13 m (2H), 2,95 s (3H), 1,38 m (2H), 1,24 m (2H), 0,85 t (3H).

c) 2-(Methylsulfinyl)benzolsulfonylisocyanat

In eine Lösung von 25,0 g N-(n-Butylamino)carbonyl-2-methylsufinylbenzolsulfonamid und 0,4 g 1,4-Diazabicyclo[2.2.2]octan in 400 ml Xylol leitet man unter Rückfluß (Trockeneiskühlung) langsam Phosgen ein, bis eine Innentemperatur von 100 °C erreicht ist. Man entfernt die Kühlung und destilliert die flüchtigen Bestandteile im Wasserstrahlvakuum bei 80 °C ab. Das verbleibende Sulfonylisocyanat wird ohne weitere Reinigung umgesetzt.

2-[N,N-(Dimethylamino)sulfonyl]benzolsulfonylisocyanat

a) N-(n-Butylamino)carbonyl-2-(N,N-dimethylaminosulfonyl)benzolsulfonamid

Zu einer Suspension von 44,2 g 2-[(N,N-Dimethylamino)sulfonyl]benzolsulfonamid (0,17 mol) (hergestellt in analoger Weise wie 2-[(N,N-Diethylamino)sulfonyl]benzolsulfonamid in US 4 310 346) in 450 ml Acetonitril tropft man bei 25 °C 18,6 g n-Butylisocyanat (0,18 mol). Nach Zugabe von 25,4 g Kaliumcarbonat (0,18 mol) rührt man 3 h unter Rückfluß. Nach Abkühlen auf 0 °C gießt man auf 400 ml Eis/Wasser, stellt durch Zugabe von konz. Salzsäure einen pH-Wert von 1 ein, saugt den gebildeten Niederschlag ab, wäscht diesen mit Wasser neutral und trocknet ihn im Wasserstrahlvakuum bei 40 °C. Man erhält so 60,0 g der Titelverbindung (99 % d.Th.) als blaßgelbes Pulver. $^1$H-NMR-Spektrum (250 MHz, $CDCl_3$, int. TMS): 8,55 br (1H), 8,30 d (1H), 8,05 d (1H), 7,7-7,9 m (1H), 6,52 t (1H), 3,17 qua (2H), 2,94 s (6H), 1,43 qui (2H), 1,25 sext (2H), 0,85 t (3H).

b) 2-[N,N-(Dimethylamino)sulfonyl]benzolsulfonylisocyanat

Der in a) erhaltene Sulfonylharnstoff wird analog zur Darstellung von 2-(Methylsulfinyl)-benzolsulfonylisocyanat in das entsprechende Sulfonylisocyanat überführt.

IV. Herstellung der Sulfonylharnstoffverbindungen I

Beispiel IV.1

N-[(4-Fluor-6-trifluormethoxy-1,3-pyrimidin-2-yl)aminocarbonyl]-2-(ethylsulfonyl)benzolsulfonamid

4,1 g (0,015 mol) 2-(Ethylsulfonyl)benzolsulfonylisocyanat in 40 ml 1,2-Dichlorethan wurden bei 25 °C unter Rühren innerhalb 15 Minuten zu 2-Amino-4-fluor-6-trifluormethoxypyrimidin in 100 ml 1,2-Dichlorethan gegeben und 12 Stunden nachgerührt. Das Reaktionsgemisch wurde im Vakuum eingeengt und der Rückstand mit Methyl-tert.-butylether verrührt, abgesaugt, gewaschen und getrocknet. Man erhielt 5,5 g (78 % d.Th.) der Titelverbindung als farblose Kristalle vom Fp. 160 °C (Zers.).
(Wirkstoffbeispiel 1.003)

Beispiel IV.2

N-[(4-Fluor-6-trifluormethoxy-1,3-pyrimidin-2-yl)aminocarbonyl]-2-(ethylsulfonyl)benzolsulfonamid-natriumsalz

0,88 g (0,0049 mol) 30 %ige Natriummethylatlösung wurden bei 0 °C unter Rühren zu einer Suspension von 2,3 g (0,0049 mol) der Verbindung aus Beispiel IV.1 gegeben und 30 Minuten bei 0 °C gerührt. Nach dem Einengen im Vakuum, Verrühren des Rückstandes mit Methyl-tert.-butylether, Waschen und Trocknen erhielt man die Titelverbindung als farblose Kristalle vom Fp. 133 °C (Zers.).
(Wirkstoffbeispiel 1.021)

Beispiel IV.3

2-[[(4-Fluor-6-trifluormethoxypyrimidin-2-yl)aminocarbonyl]aminosulfonyl]benzolsulfonsäure(N,N-dimethyl)-amid

Eine Lösung von 4,0 g 2-Amino-4-fluor-6-trifluormethoxypyrimidin (20 mmol) in 30 ml Methylenchlorid wird bei 25 °C mit 5,9 g 2-(Dimethylamino)sulfonylbenzolsulfonylisocyanat (20 mmol) versetzt. Man rührt 16

h bei 25°C nach, saugt das abgeschiedene Produkt ab, wäscht mit wenig Ether und trocknet im Wasserstrahlvakuum bei 50°C. Man erhält so 2,1 g der Titelverbindung (22 % d.Th.) mit Fp. 167-169°C. Aus der Mutterlauge kann weiteres Produkt isoliert werden.
(Wirkstoffbeispiel 15.001)

Beispiel IV.4

N-[(4-Methoxy-6-trifluormethoxypyrimidin-2-yl)aminocarbonyl]-2-methylsulfinylbenzolsulfonamid

Eine Lösung von 4,0 g 2-Amino-4-methoxy-6-trifluormethoxypyrimidin (19 mmol) in 30 ml Methylenchlorid wird bei 25°C mit 4,7 g 2-(Methylsulfinyl)benzolsulfonylisocyanat (19 mmol) versetzt. Man rührt 16 h bei 25°C nach, saugt das abgeschiedene Produkt ab, wäscht mit wenig Ether und trocknet im Wasserstrahlvakuum bei 50°C. Man erhält so 0,9 g der Titelverbindung (10 % d.Th.) mit Fp. 110-116°C. Aus der Mutterlauge kann weiteres Produkt isoliert werden.
(Wirkstoffbeispiel 14.007)

Tabelle 1:

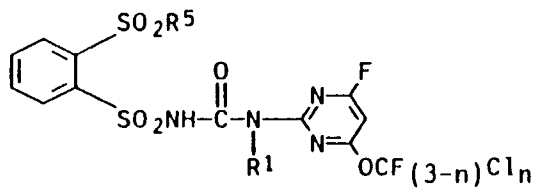

| Nr. | $R^1$ | $R^5$ | n | Fp (°C) |
|---|---|---|---|---|
| 1.001 | H | $CH_3$ | 0 | |
| 1.002 | $CH_3$ | $CH_3$ | 0 | |
| 1.003 | H | $CH_2CH_3$ | 0 | 160 Zers. |
| 1.004 | $CH_3$ | $CH_2CH_3$ | 0 | |
| 1.005 | H | $(CH_2)_2CH_3$ | 0 | 164 Zers. |
| 1.006 | $CH_3$ | $(CH_2)_2CH_3$ | 0 | |
| 1.007 | H | $CH(CH_3)_2$ | 0 | 198-199 |
| 1.008 | H | $CH_2-CH=CH_2$ | 0 | |
| 1.009 | H | $CH_2-CH=CH-CH_3$ | 0 | |
| 1.010 | H | $CH_2-C\equiv C-CH_3$ | 0 | |
| 1.011 | H | $(CH_2)_2Cl$ | 0 | |
| 1.012 | $CH_3$ | $(CH_2)_2Cl$ | 0 | |

Tabelle 1: (Fortsetzung)

| Nr. | $R^1$ | $R^5$ | n | Fp ($^oC$) | |
|---|---|---|---|---|---|
| 1.013 | H | $(CH_2)_2OCH_3$ | 0 | | |
| 1.014 | H | $(CH_2)_2O(CH_2)_2OCH_3$ | 0 | | |
| 1.015 | H | Cyclopentyl | 0 | | |
| 1.016 | H | Cyclohexyl | 0 | | |
| 1.017 | H | $CH_2CF_3$ | 0 | | |
| 1.018 | H | $CH=CH_2$ | 0 | | |
| 1.019 | H | $CH_3$ | 0 | | Na-salz |
| 1.020 | $CH_3$ | $CH_3$ | 0 | | Na-salz |
| 1.021 | H | $CH_2CH_3$ | 0 | 133 Zers. | Na-salz |
| 1.022 | $CH_3$ | $CH_2CH_3$ | 0 | | Na-salz |
| 1.023 | H | $(CH_2)_2CH_3$ | 0 | 150 Zers. | Na-salz |
| 1.024 | H | $(CH_2)_2Cl$ | 0 | | Na-salz |
| 1.025 | H | $CH(CH_3)_2$ | 0 | 169 Zers. | Na-salz |

Tabelle 2:

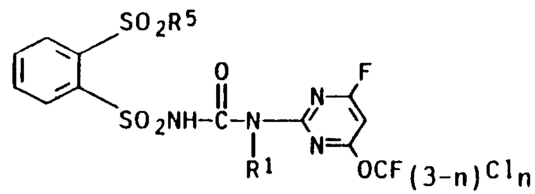

| Nr. | $R^1$ | $R^5$ | n | Fp (°C) |
|---|---|---|---|---|
| 2.001 | H | $CH_3$ | 1 | |
| 2.002 | $CH_3$ | $CH_3$ | 1 | |
| 2.003 | H | $CH_2CH_3$ | 1 | |
| 2.004 | $CH_3$ | $CH_2CH_3$ | 1 | |
| 2.005 | H | $(CH_2)_2CH_3$ | 1 | 169-172 Zers. |
| 2.006 | $CH_3$ | $(CH_2)_2CH_3$ | 1 | |
| 2.007 | H | $CH(CH_3)_2$ | 1 | |
| 2.008 | H | $CH_2-CH=CH_2$ | 1 | |
| 2.009 | H | $CH_2-CH=CH-CH_3$ | 1 | |
| 2.010 | H | $CH_2-C\equiv C-CH_3$ | 1 | |
| 2.011 | H | $(CH_2)_2Cl$ | 1 | |
| 2.012 | $CH_3$ | $(CH_2)_2Cl$ | 1 | |

Tabelle 2: (Fortsetzung)

| Nr. | $R^1$ | $R^5$ | n | Fp ($^oC$) | |
|---|---|---|---|---|---|
| 2.013 | H | $(CH_2)_2OCH_3$ | 1 | | |
| 2.014 | H | $(CH_2)_2O(CH_2)_2OCH_3$ | 1 | | |
| 2.015 | H | Cyclopentyl | 1 | | |
| 2.016 | H | Cyclohexyl | 1 | | |
| 2.017 | H | $CH_2CF_3$ | 1 | | |
| 2.018 | H | $CH=CH_2$ | 1 | | |
| 2.019 | H | $CH_3$ | 1 | | Na-salz |
| 2.020 | $CH_3$ | $CH_3$ | 1 | | Na-salz |
| 2.021 | H | $CH_2CH_3$ | 1 | | Na-salz |
| 2.022 | $CH_3$ | $CH_2CH_3$ | 1 | | Na-salz |
| 2.023 | H | $(CH_2)_2CH_3$ | 1 | 138 Zers. | Na-salz |
| 2.024 | H | $(CH_2)_2Cl$ | 1 | | Na-salz |

Tabelle 3:

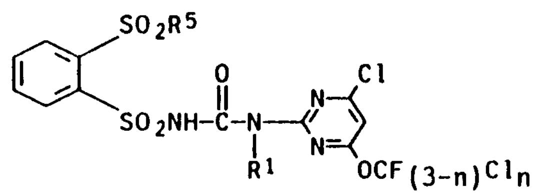

| Nr. | $R^1$ | $R^5$ | n | Fp (°C) |
|---|---|---|---|---|
| 3.001 | H | $CH_3$ | 0 | |
| 3.002 | $CH_3$ | $CH_3$ | 0 | |
| 3.003 | H | $CH_2CH_3$ | 0 | 173-178 |
| 3.004 | $CH_3$ | $CH_2CH_3$ | 0 | |
| 3.005 | H | $(CH_2)_2CH_3$ | 0 | |
| 3.006 | $CH_3$ | $(CH_2)_2CH_3$ | 0 | |
| 3.007 | H | $CH(CH_3)_2$ | 0 | |
| 3.008 | H | $CH_2-CH=CH_2$ | 0 | |
| 3.009 | H | $CH_2-CH=CH-CH_3$ | 0 | |
| 3.010 | H | $CH_2-C\equiv C-CH_3$ | 0 | |
| 3.011 | H | $(CH_2)_2Cl$ | 0 | |
| 3.012 | $CH_3$ | $(CH_2)_2Cl$ | 0 | |

Tabelle 3: (Fortsetzung)

| Nr. | $R^1$ | $R^5$ | n | Fp ($^oC$) | |
|---|---|---|---|---|---|
| 3.013 | H | $(CH_2)_2OCH_3$ | 0 | | |
| 3.014 | H | $(CH_2)_2O(CH_2)_2OCH_3$ | 0 | | |
| 3.015 | H | Cyclopentyl | 0 | | |
| 3.016 | H | Cyclohexyl | 0 | | |
| 3.017 | H | $CH_2CF_3$ | 0 | | |
| 3.018 | H | $CH{=}CH_2$ | 0 | | |
| 3.019 | H | $CH_3$ | 0 | | Na-salz |
| 3.020 | $CH_3$ | $CH_3$ | 0 | | Na-salz |
| 3.021 | H | $CH_2CH_3$ | 0 | 130 Zers. | Na-salz |
| 3.022 | $CH_3$ | $CH_2CH_3$ | 0 | | Na-salz |
| 3.023 | H | $(CH_2)_2CH_3$ | 0 | | Na-salz |
| 3.024 | H | $(CH_2)_2Cl$ | 0 | | Na-salz |

Tabelle 4:

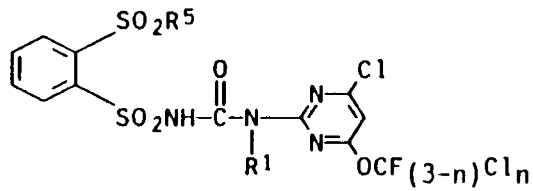

| Nr. | $R^1$ | $R^5$ | n | Fp (°C) |
|---|---|---|---|---|
| 4.001 | H | $CH_3$ | 1 | |
| 4.002 | $CH_3$ | $CH_3$ | 1 | |
| 4.003 | H | $CH_2CH_3$ | 1 | |
| 4.004 | $CH_3$ | $CH_2CH_3$ | 1 | |
| 4.005 | H | $(CH_2)_2CH_3$ | 1 | |
| 4.006 | $CH_3$ | $(CH_2)_2CH_3$ | 1 | |
| 4.007 | H | $CH(CH_3)_2$ | 1 | |
| 4.008 | H | $CH_2-CH=CH_2$ | 1 | |
| 4.009 | H | $CH_2-CH=CH-CH_3$ | 1 | |
| 4.010 | H | $CH_2-C \equiv C-CH_3$ | 1 | |
| 4.011 | H | $(CH_2)_2Cl$ | 1 | |
| 4.012 | $CH_3$ | $(CH_2)_2Cl$ | 1 | |

Tabelle 4: (Fortsetzung)

| Nr. | $R^1$ | $R^5$ | n | Fp (°C) |
|---|---|---|---|---|
| 4.013 | H | $(CH_2)_2OCH_3$ | 1 | |
| 4.014 | H | $(CH_2)_2O(CH_2)_2OCH_3$ | 1 | |
| 4.015 | H | Cyclopentyl | 1 | |
| 4.016 | H | Cyclohexyl | 1 | |
| 4.017 | H | $CH_2CF_3$ | 1 | |
| 4.018 | H | $CH=CH_2$ | 1 | |
| 4.019 | H | $CH_3$ | 1 | Na-salz |
| 4.020 | $CH_3$ | $CH_3$ | 1 | Na-salz |
| 4.021 | H | $CH_2CH_3$ | 1 | Na-salz |
| 4.022 | $CH_3$ | $CH_2CH_3$ | 1 | Na-salz |
| 4.023 | H | $(CH_2)_2CH_3$ | 1 | Na-salz |
| 4.024 | H | $(CH_2)_2Cl$ | 1 | Na-salz |

Tabelle 5:

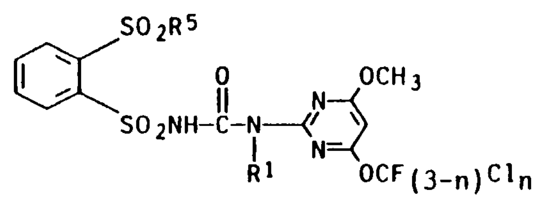

| Nr. | $R^1$ | $R^5$ | n | Fp ($^oC$) |
|---|---|---|---|---|
| 5.001 | H | $CH_3$ | 0 | 193-196 |
| 5.002 | $CH_3$ | $CH_3$ | 0 | |
| 5.003 | H | $CH_2CH_3$ | 0 | 154-157 |
| 5.004 | $CH_3$ | $CH_2CH_3$ | 0 | |
| 5.005 | H | $(CH_2)_2CH_3$ | 0 | 189-192 Zers. |
| 5.006 | $CH_3$ | $(CH_2)_2CH_3$ | 0 | |
| 5.007 | H | $CH(CH_3)_2$ | 0 | 138-143 Zers. |
| 5.008 | H | $CH_2-CH=CH_2$ | 0 | |
| 5.009 | H | $CH_2-CH=CH-CH_3$ | 0 | |
| 5.010 | H | $CH_2-C\equiv C-CH_3$ | 0 | |
| 5.011 | H | $(CH_2)_2Cl$ | 0 | |
| 5.012 | $CH_3$ | $(CH_2)_2Cl$ | 0 | |

Tabelle 5: (Fortsetzung)

| Nr. | $R^1$ | $R^5$ | n | Fp (°C) | |
|---|---|---|---|---|---|
| 5.013 | H | $(CH_2)_2OCH_3$ | 0 | | |
| 5.014 | H | $(CH_2)_2O(CH_2)_2OCH_3$ | 0 | | |
| 5.015 | H | Cyclopentyl | 0 | | |
| 5.016 | H | Cyclohexyl | 0 | | |
| 5.017 | H | $CH_2CF_3$ | 0 | | |
| 5.018 | H | $CH{=}CH_2$ | 0 | | |
| 5.019 | H | $CH_3$ | 0 | | Na-salz, |
| 5.020 | $CH_3$ | $CH_3$ | 0 | | Na-salz |
| 5.021 | H | $CH_2CH_3$ | 0 | 170 Zers. | Na-salz, |
| 5.022 | $CH_3$ | $CH_2CH_3$ | 0 | | Na-salz |
| 5.023 | H | $(CH_2)_2CH_3$ | 0 | 167 Zers. | Na-salz |
| 5.024 | H | $(CH_2)_2Cl$ | 0 | | Na-salz |

Tabelle 6:

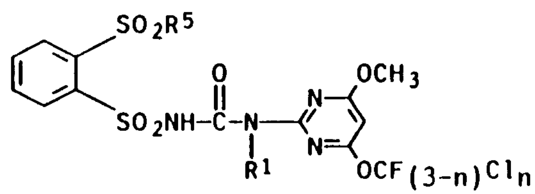

| Nr. | $R^1$ | $R^5$ | n | Fp (°C) |
|---|---|---|---|---|
| 6.001 | H | $CH_3$ | 1 | 178-180 |
| 6.002 | $CH_3$ | $CH_3$ | 1 | |
| 6.003 | H | $CH_2CH_3$ | 1 | |
| 6.004 | $CH_3$ | $CH_2CH_3$ | 1 | |
| 6.005 | H | $(CH_2)_2CH_3$ | 1 | 172-177 Zers. |
| 6.006 | $CH_3$ | $(CH_2)_2CH_3$ | 1 | |
| 6.007 | H | $CH(CH_3)_2$ | 1 | |
| 6.008 | H | $CH_2$-CH=$CH_2$ | 1 | |
| 6.009 | H | $CH_2$-CH=CH-$CH_3$ | 1 | |
| 6.010 | H | $CH_2$-C≡C-$CH_3$ | 1 | |
| 6.011 | H | $(CH_2)_2Cl$ | 1 | |
| 6.012 | $CH_3$ | $(CH_2)_2Cl$ | 1 | |

Tabelle 6: (Fortsetzung)

| Nr. | $R^1$ | $R^5$ | n | Fp ($^oC$) | |
|---|---|---|---|---|---|
| 6.013 | H | $(CH_2)_2OCH_3$ | 1 | | |
| 6.014 | H | $(CH_2)_2O(CH_2)_2OCH_3$ | 1 | | |
| 6.015 | H | Cyclopentyl | 1 | | |
| 6.016 | H | Cyclohexyl | 1 | | |
| 6.017 | H | $CH_2CF_3$ | 1 | | |
| 6.018 | H | $CH=CH_2$ | 1 | | |
| 6.019 | H | $CH_3$ | 1 | | Na-salz |
| 6.020 | $CH_3$ | $CH_3$ | 1 | | Na-salz |
| 6.021 | H | $CH_2CH_3$ | 1 | | Na-salz |
| 6.022 | $CH_3$ | $CH_2CH_3$ | 1 | | Na-salz |
| 6.023 | H | $(CH_2)_2CH_3$ | 1 | 181 Zers. | Na-salz |
| 6.024 | H | $(CH_2)_2Cl$ | 1 | | Na-salz |

Tabelle 7:

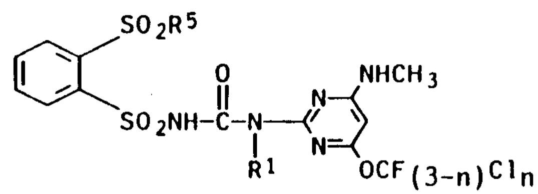

| Nr. | $R^1$ | $R^5$ | n | Fp (°C) |
|---|---|---|---|---|
| 7.001 | H | $CH_3$ | 0 | |
| 7.002 | $CH_3$ | $CH_3$ | 0 | |
| 7.003 | H | $CH_2CH_3$ | 0 | 127 |
| 7.004 | $CH_3$ | $CH_2CH_3$ | 0 | |
| 7.005 | H | $(CH_2)_2CH_3$ | 0 | 154 |
| 7.006 | $CH_3$ | $(CH_2)_2CH_3$ | 0 | |
| 7.007 | H | $CH(CH_3)_2$ | 0 | |
| 7.008 | H | $CH_2-CH=CH_2$ | 0 | |
| 7.009 | H | $CH_2-CH=CH-CH_3$ | 0 | |
| 7.010 | H | $CH_2-C\equiv C-CH_3$ | 0 | |
| 7.011 | H | $(CH_2)_2Cl$ | 0 | |
| 7.012 | $CH_3$ | $(CH_2)_2Cl$ | 0 | |

Tabelle 7: (Fortsetzung)

| Nr. | $R^1$ | $R^5$ | n | Fp (°C) | |
|---|---|---|---|---|---|
| 7.013 | H | $(CH_2)_2OCH_3$ | 0 | | |
| 7.014 | H | $(CH_2)_2O(CH_2)_2OCH_3$ | 0 | | |
| 7.015 | H | Cyclopentyl | 0 | | |
| 7.016 | H | Cyclohexyl | 0 | | |
| 7.017 | H | $CH_2CF_3$ | 0 | | |
| 7.018 | H | $CH=CH_2$ | 0 | | |
| 7.019 | H | $CH_3$ | 0 | | Na-salz |
| 7.020 | $CH_3$ | $CH_3$ | 0 | | Na-salz |
| 7.021 | H | $CH_2CH_3$ | 0 | 192 Zers. | Na-salz |
| 7.022 | $CH_3$ | $CH_2CH_3$ | 0 | | Na-salz |
| 7.023 | H | $(CH_2)_2CH_3$ | 0 | 181 Zers. | Na-salz |
| 7.024 | H | $(CH_2)_2Cl$ | 0 | | Na-salz |

Tabelle 8:

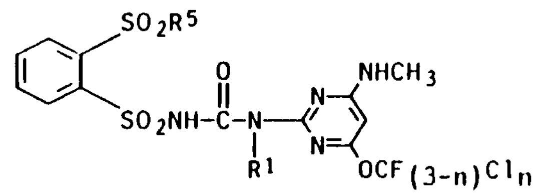

| Nr. | $R^1$ | $R^5$ | n | Fp (°C) |
|---|---|---|---|---|
| 8.001 | H | $CH_3$ | 1 | |
| 8.002 | $CH_3$ | $CH_3$ | 1 | |
| 8.003 | H | $CH_2CH_3$ | 1 | |
| 8.004 | $CH_3$ | $CH_2CH_3$ | 1 | |
| 8.005 | H | $(CH_2)_2CH_3$ | 1 | |
| 8.006 | $CH_3$ | $(CH_2)_2CH_3$ | 1 | |
| 8.007 | H | $CH(CH_3)_2$ | 1 | |
| 8.008 | H | $CH_2-CH=CH_2$ | 1 | |
| 8.009 | H | $CH_2-CH=CH-CH_3$ | 1 | |
| 8.010 | H | $CH_2-C\equiv C-CH_3$ | 1 | |
| 8.011 | H | $(CH_2)_2Cl$ | 1 | |
| 8.012 | $CH_3$ | $(CH_2)_2Cl$ | 1 | |

Tabelle 8: (Fortsetzung)

| Nr. | $R^1$ | $R^5$ | n | Fp (°C) |
|---|---|---|---|---|
| 8.013 | H | $(CH_2)_2OCH_3$ | 1 | |
| 8.014 | H | $(CH_2)_2O(CH_2)_2OCH_3$ | 1 | |
| 8.015 | H | Cyclopentyl | 1 | |
| 8.016 | H | Cyclohexyl | 1 | |
| 8.017 | H | $CH_2CF_3$ | 1 | |
| 8.018 | H | $CH=CH_2$ | 1 | |
| 8.019 | H | $CH_3$ | 1 | Na-salz |
| 8.020 | $CH_3$ | $CH_3$ | 1 | Na-salz |
| 8.021 | H | $CH_2CH_3$ | 1 | Na-salz |
| 8.022 | $CH_3$ | $CH_2CH_3$ | 1 | Na-salz |
| 8.023 | H | $(CH_2)_2CH_3$ | 1 | Na-salz |
| 8.024 | H | $(CH_2)_2Cl$ | 1 | Na-salz |

Tabelle 9:

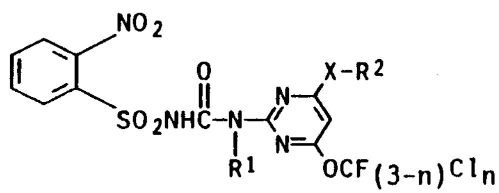

| Nr. | $R^1$ | X | $R^2$ | n | Fp (°C) | |
|---|---|---|---|---|---|---|
| 9.001 | H | - | F | 0 | 148-154 | |
| 9.002 | H | - | Cl | 0 | | |
| 9.003 | $CH_3$ | - | F | 0 | | |
| 9.004 | $CH_3$ | - | Cl | 0 | | |
| 9.005 | H | - | F | 1 | 153-160 Zers. | |
| 9.006 | H | - | Cl | 1 | | |
| 9.007 | $CH_3$ | - | F | 1 | | |
| 9.008 | $CH_3$ | - | Cl | 1 | | |
| 9.009 | H | - | F | 0 | 140 Zers. | Na-Salz |
| 9.010 | H | - | Cl | 0 | | Na-Salz |
| 9.011 | H | O | $CH_3$ | 0 | 189-191 | |
| 9.012 | H | O | $CH_3$ | 1 | 149-152 | |
| 9.013 | $CH_3$ | O | $CH_3$ | 0 | 93-96 | |
| 9.014 | $CH_3$ | O | $CH_3$ | 1 | | |

Tabelle 9: (Fortsetzung)

| Nr. | $R^1$ | X | $R^2$ | n | Fp (°C) |
|---|---|---|---|---|---|
| 9.015 | $CH_3$ | O | $CH_3$ | 0 | 192 Zers. Na-Salz |
| 9.016 | $CH_3$ | O | $CH_3$ | 1 | Na-Salz |
| 9.017 | H | NH | $CH_3$ | 0 | 211 Zers. |
| 9.018 | H | NH | $CH_3$ | 1 | |
| 9.019 | H | $NCH_3$ | $CH_3$ | 0 | 193-196 Zers. |
| 9.020 | H | $NCH_3$ | $CH_3$ | 1 | |
| 9.021 | $CH_3$ | NH | $CH_3$ | 0 | |
| 9.022 | H | O | $CH_3$ | 0 | 155 Zers. Na-Salz |
| 9.023 | H | - | F | 1 | 130 Zers. Na-Salz |
| 9.024 | H | $NCH_3$ | $CH_3$ | 0 | |
| 9.025 | H | $NCH_3$ | $CH_3$ | 0 | 152-158 Na-Salz |
| 9.026 | H | NH | $CH_3$ | 0 | 165 Zers. Na-Salz |
| 9.027 | H | O | $CH_3$ | 1 | 151 Zers. Na-Salz |

Tabelle 10:

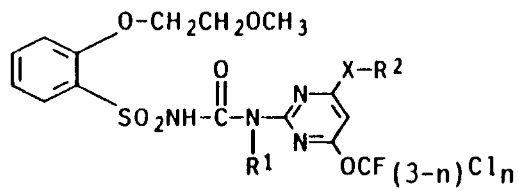

| Nr. | $R^1$ | X | $R^2$ | n | Fp (°C) | |
|---|---|---|---|---|---|---|
| 10.001 | H | - | F | 0 | 132-135 | |
| 10.002 | H | - | Cl | 0 | | |
| 10.003 | H | - | F | 1 | 125-128 | |
| 10.004 | H | - | Cl | 1 | | |
| 10.005 | H | O | $CH_3$ | 0 | 131-135 | |
| 10.006 | H | O | $CH_3$ | 1 | 148-150 | |
| 10.007 | H | NH | $CH_3$ | 0 | | |
| 10.008 | H | NH | $CH_3$ | 1 | | |
| 10.009 | $CH_3$ | NH | $CH_3$ | 0 | | |
| 10.010 | $CH_3$ | NH | $CH_3$ | 1 | | |
| 10.011 | H | O | $CH_3$ | 0 | | Na-Salz |
| 10.012 | H | - | F | 0 | 175-180 Zers. | Na-Salz |
| 10.013 | H | O | $CH_3$ | 1 | 255 Zers. | Na-Salz |
| 10.014 | H | O | $C_2H_5$ | 0 | 142-144 | |

Tabelle 10: (Fortsetzung)

| Nr. | $R^1$ | X | $R^2$ | n | Fp (°C) | |
|---|---|---|---|---|---|---|
| 10.015 | H | O | $C_2H_5$ | 1 | 121-123 | |
| 10.016 | H | NH | $CH_3$ | 0 | | Na-Salz |
| 10.017 | H | NH | $CH_3$ | 1 | | Na-Salz |
| 10.018 | H | $NCH_3$ | $CH_3$ | 0 | | |
| 10.019 | H | $NCH_3$ | $CH_3$ | 0 | | Na-Salz |
| 10.020 | H | $NCH_3$ | $CH_3$ | 1 | | |
| 10.021 | H | $NCH_3$ | $CH_3$ | 1 | | Na-Salz |

Tabelle 11:

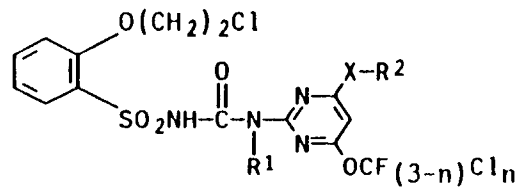

| Nr. | $R^1$ | X | $R^2$ | n | FP (°C) | |
|---|---|---|---|---|---|---|
| 11.001 | H | - | F | 0 | | |
| 11.002 | H | - | Cl | 0 | | |
| 11.003 | H | - | F | 1 | | |
| 11.004 | H | - | Cl | 1 | | |
| 11.005 | H | O | $CH_3$ | 0 | 147-150 | |
| 11.006 | H | O | $CH_3$ | 1 | 141-143 | |
| 11.007 | H | NH | $CH_3$ | 0 | | |
| 11.008 | H | NH | $CH_3$ | 1 | | |
| 11.009 | $CH_3$ | NH | $CH_3$ | 0 | | |
| 11.010 | $CH_3$ | NH | $CH_3$ | 1 | | |
| 11.011 | H | O | $CH_3$ | 0 | | Na-Salz |
| 11.012 | H | - | F | 0 | | Na-Salz |
| 11.013 | H | O | $CH_3$ | 1 | | Na-Salz |
| 11.014 | H | NH | $CH_3$ | 0 | | Na-Salz |
| 11.015 | H | NH | $CH_3$ | 1 | | Na-Salz |

Tabelle 11 (Fortsetzung):

| Nr. | $R^1$ | X | $R^2$ | n | FP (°C) | |
|---|---|---|---|---|---|---|
| 11.016 | H | $NCH_3$ | $CH_3$ | 0 | 203-205 | |
| 11.017 | H | $NCH_3$ | $CH_3$ | 0 | 135-140 | Na-Salz |
| 11.018 | H | $NCH_3$ | $CH_3$ | 1 | | |
| 11.019 | H | $NCH_3$ | $CH_3$ | 1 | | Na-Salz |

Tabelle 12:

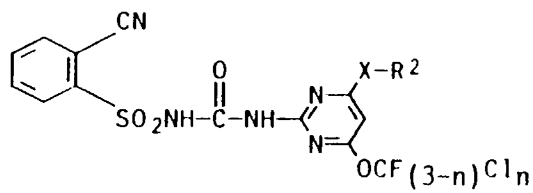

| Nr. | $R^1$ | X | $R^2$ | n | FP (°C) |
|---|---|---|---|---|---|
| 12.001 | H | - | F | 0 | |
| 12.002 | H | - | Cl | 0 | |
| 12.003 | H | - | F | 1 | |
| 12.004 | H | - | Cl | 1 | |
| 12.005 | H | O | $CH_3$ | 0 | |
| 12.006 | H | O | $CH_3$ | 1 | |
| 12.007 | H | NH | $CH_3$ | 0 | |
| 12.008 | H | NH | $CH_3$ | 1 | |
| 12.009 | $CH_3$ | NH | $CH_3$ | 0 | |
| 12.010 | $CH_3$ | NH | $CH_3$ | 1 | Na-Salz |
| 12.011 | H | O | $CH_3$ | 0 | Na-Salz |
| 12.012 | H | - | F | 0 | |
| 12.013 | H | O | $CH_3$ | 1 | Na-Salz |
| 12.014 | H | NH | $CH_3$ | 0 | Na-Salz |
| 12.015 | H | NH | $CH_3$ | 1 | Na-Salz |

Tabelle 12 (Fortsetzung):

| Nr. | $R^1$ | X | $R^2$ | n | FP (°C) |
|---|---|---|---|---|---|
| 12.016 | H | $NCH_3$ | $CH_3$ | 0 | |
| 12.017 | H | $NCH_3$ | $CH_3$ | 0 | Na-Salz |
| 12.018 | H | $NCH_3$ | $CH_3$ | 1 | |
| 12.019 | H | $NCH_3$ | $CH_3$ | 1 | Na-Salz |

Tabelle 13:

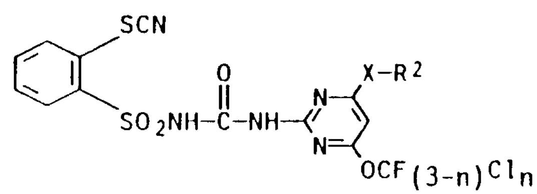

| Nr. | $R^1$ | X | $R^2$ | n | FP (°C) |
|---|---|---|---|---|---|
| 13.001 | H | - | F | 0 | |
| 13.002 | H | - | Cl | 0 | |
| 13.003 | H | - | F | 1 | |
| 13.004 | H | - | Cl | 1 | |
| 13.005 | H | O | $CH_3$ | 0 | |
| 13.006 | H | O | $CH_3$ | 1 | |
| 13.007 | H | NH | $CH_3$ | 0 | |
| 13.008 | H | NH | $CH_3$ | 1 | |
| 13.009 | $CH_3$ | NH | $CH_3$ | 0 | |
| 13.010 | $CH_3$ | NH | $CH_3$ | 1 | Na-Salz |
| 13.011 | H | O | $CH_3$ | 0 | Na-Salz |
| 13.012 | H | - | F | 0 | |
| 13.013 | H | O | $CH_3$ | 1 | Na-Salz |
| 13.014 | H | NH | $CH_3$ | 0 | Na-Salz |
| 13.015 | H | NH | $CH_3$ | 1 | Na-Salz |

Tabelle 13 (Fortsetzung):

| Nr. | $R^1$ | X | $R^2$ | n | FP (°C) |
|---|---|---|---|---|---|
| 13.016 | H | $NCH_3$ | $CH_3$ | 0 | |
| 13.017 | H | $NCH_3$ | $CH_3$ | 0 | Na-Salz |
| 13.018 | H | $NCH_3$ | $CH_3$ | 1 | |
| 13.019 | H | $NCH_3$ | $CH_3$ | 1 | Na-Salz |

Tabelle 14:

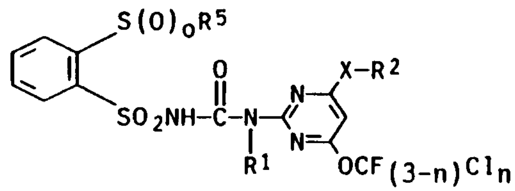

| Nr. | $R^1$ | R5 | X | $R^2$ | n | O | FP (°C) | |
|---|---|---|---|---|---|---|---|---|
| 14.001 | H | $CH_3$ | - | F | 0 | 1 | | |
| 14.002 | H | $CH_3$ | - | F | 0 | 1 | | Na-Salz |
| 14.003 | H | $C_2H_5$ | - | F | 0 | 1 | | |
| 14.004 | H | $C_2H_5$ | - | F | 0 | 1 | | Na-Salz |
| 14.005 | H | $n-C_3H_7$ | - | F | 0 | 1 | | |
| 14.006 | H | $n-C_3H_7$ | - | F | 0 | 1 | | Na-Salz |
| 14.007 | H | $CH_3$ | O | $CH_3$ | 0 | 1 | 110-116 | |
| 14.008 | H | $CH_3$ | O | $CH_3$ | 0 | 1 | 150-151 (Zers.) | Na-Salz |
| 14.009 | H | $C_2H_5$ | O | $CH_3$ | 0 | 0 | | |
| 14.010 | H | $C_2H_5$ | O | $CH_3$ | 0 | 0 | | Na-Salz |
| 14.011 | H | $n-C_3H_7$ | O | $CH_3$ | 0 | 1 | | |
| 14.012 | H | $n-C_3H_7$ | O | $CH_3$ | 0 | 1 | | Na-Salz |
| 14.013 | H | $i-C_3H_7$ | O | $CH_3$ | 0 | 1 | | |
| 14.014 | H | $i-C_3H_7$ | O | $CH_3$ | 0 | 1 | | Na-Salz |
| 14.015 | H | $C_2H_5$ | O | $CH_3$ | 0 | 1 | | |

Tabelle 14: (Fortsetzung)

| Nr. | $R^1$ | R5 | X | $R^2$ | n | O | FP (°C) | |
|---|---|---|---|---|---|---|---|---|
| 14.016 | H | $C_2H_5$ | O | $CH_3$ | 0 | 1 | | Na-Salz |
| 14.017 | H | $C_2H_5$ | O | $CH_3$ | 1 | 1 | | |
| 14.018 | H | $C_2H_5$ | O | $CH_3$ | 1 | 1 | | Na-Salz |
| 14.019 | H | $n-C_3H_7$ | O | $CH_3$ | 1 | 1 | | |
| 14.020 | H | $n-C_3H_7$ | O | $CH_3$ | 1 | 1 | | Na-Salz |
| 14.021 | $CH_3$ | $C_2H_5$ | O | $CH_3$ | 0 | 1 | | |
| 14.022 | H | $C_2H_5$ | NH | $CH_3$ | 0 | 1 | | |
| 14.023 | H | $C_2H_5$ | NH | $CH_3$ | 0 | 1 | | Na-Salz |
| 14.024 | H | $C_2H_5$ | $NCH_3$ | $CH_3$ | 0 | 1 | | |
| 14.025 | H | $C_2H_5$ | $NCH_3$ | $CH_3$ | 0 | 2 | 198-202 | |
| 14.026 | H | $C_2H_5$ | $NCH_3$ | $CH_3$ | 0 | 2 | 169 Zers. | Na-Salz |
| 14.027 | H | $CH_3$ | O | $CH_3$ | 0 | 0 | 141-144 | |

Tabelle 15:

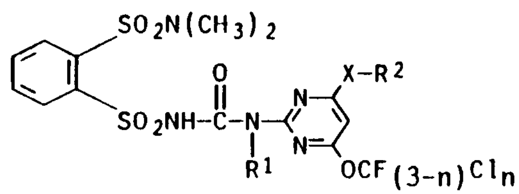

| Nr. | $R^1$ | X | $R^2$ | n | FP (°C) |
|---|---|---|---|---|---|
| 15.001 | H | - | F | | 167-169 |
| 15.002 | H | - | Cl | 0 | |
| 15.003 | H | - | F | 1 | |
| 15.004 | H | - | Cl | 1 | |
| 15.005 | H | O | $CH_3$ | 0 | 160-163 |
| 15.006 | H | O | $CH_3$ | 1 | 145-150 |
| 15.007 | H | NH | $CH_3$ | 0 | |
| 15.008 | H | NH | $CH_3$ | 1 | |
| 15.009 | $CH_3$ | NH | $CH_3$ | 0 | |
| 15.010 | $CH_3$ | NH | $CH_3$ | 1 | |
| 15.011 | H | O | $CH_3$ | 0 | 165-177 (Zers.) Na-Salz |
| 15.012 | H | - | F | 0 | 127-133 (Zers.) Na-Salz |
| 15.013 | H | O | $CH_3$ | 1 | Na-Salz |
| 15.014 | H | NH | $CH_3$ | 0 | Na-Salz |
| 15.015 | H | NH | $CH_3$ | 1 | Na-Salz |

Tabelle 15: (Fortsetzung)

| Nr. | $R^1$ | X | $R^2$ | n | FP (°C) | |
|---|---|---|---|---|---|---|
| 15.016 | H | $NCH_3$ | $CH_3$ | 0 | | |
| 15.017 | H | $NCH_3$ | $CH_3$ | 0 | | Na-Salz |
| 15.018 | H | $NCH_3$ | $CH_3$ | 1 | | |
| 15.019 | H | $NCH_3$ | $CH_3$ | 1 | | Na-Salz |
| 15.020 | H | O | $CH_3$ | 0 | 178-186 (Zers.) | K-Salz |

Tabelle 16:

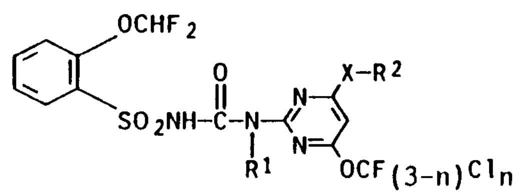

| Nr. | $R^1$ | X | $R^2$ | n | Fp (°C) | |
|---|---|---|---|---|---|---|
| 16.001 | H | - | F | 0 | | |
| 16.002 | H | - | Cl | 0 | | |
| 16.003 | H | - | F | 1 | | |
| 16.004 | H | - | Cl | 1 | | |
| 16.005 | H | O | $CH_3$ | 0 | 99-100 | |
| 16.006 | H | O | $CH_3$ | 1 | | |
| 16.007 | H | NH | $CH_3$ | 0 | | |
| 16.008 | H | NH | $CH_3$ | 1 | | |
| 16.009 | $CH_3$ | NH | $CH_3$ | 0 | | |
| 16.010 | $CH_3$ | NH | $CH_3$ | 1 | | Na-Salz |
| 16.011 | H | O | $CH_3$ | 0 | 145-152 | Na-Salz |
| 16.012 | H | - | F | 0 | | |
| 16.013 | H | O | $CH_3$ | 1 | | Na-Salz |
| 16.014 | H | NH | $CH_3$ | 0 | | Na-Salz |
| 16.015 | H | NH | $CH_3$ | 1 | | Na-Salz |

Tabelle 16 (Fortsetzung):

| Nr. | $R^1$ | X | $R^2$ | n | Fp (°C) |
|---|---|---|---|---|---|
| 16.016 | H | $NCH_3$ | $CH_3$ | 0 | |
| 16.017 | H | $NCH_3$ | $CH_3$ | 0 | Na-Salz |
| 16.018 | H | $NCH_3$ | $CH_3$ | 1 | |
| 16.019 | H | $NCH_3$ | $CH_3$ | 1 | Na-Salz |

Tabelle 17:

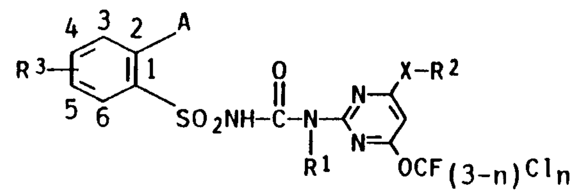

| Nr. | A | $R^3$ | $R^1$ | X | $R^2$ | n | Fp (°C) | |
|---|---|---|---|---|---|---|---|---|
| 17.001 | $OCH_3$ | H | H | O | $CH_3$ | 0 | 125-129 | |
| 17.002 | $OCH_3$ | H | H | O | $CH_3$ | 0 | 163 Zers. | Na-Salz |
| 17.003 | $CH_2OCH_3$ | H | H | O | $CH_3$ | 0 | 135 | |
| 17.004 | $N(CH_3)SO_2CH_3$ | H | H | O | $CH_3$ | 0 | 214-216 | |
| 17.005 | $N(CH_3)SO_2CH_3$ | H | H | O | $CH_3$ | 0 | 171 Zers. | Na-Salz |
| 17.006 | $OCH_3$ | 5-Cl | H | O | $CH_3$ | 0 | | |
| 17.007 | $SO_2C_2H_5$ | 5-Cl | H | O | $CH_3$ | 0 | 152-156 | |
| 17.008 | $SO_2C_2H_5$ | 5-Cl | H | O | $CH_3$ | 1 | 143-148 | |
| 17.009 | $SO_2C_2H_5$ | 5-$OCH_3$ | H | O | $CH_3$ | 0 | | |
| 17.010 | $SO_2C_2H_5$ | 5-$OCH_3$ | H | O | $CH_3$ | 1 | | |
| 17.011 | $OCH_2CF_3$ | 5-$OCH_2CF_3$ | H | O | $CH_3$ | 0 | 152-155 | |
| 17.012 | $OCF_2CF_2H$ | H | H | O | $CH_3$ | 0 | 151-153 | |
| 17.013 | $OCF_2CF_2H$ | H | H | O | $CH_3$ | 0 | 149 Zers. | Na-Salz |
| 17.014 | $OCF_2CFHCl$ | H | H | O | $CH_3$ | 0 | 145-147 | |
| 17.015 | $OCF_2CFHCl$ | H | H | O | $CH_3$ | 0 | 155 Zers. | Na-Salz |

Tabelle 17: (Fortsetzung)

| Nr. | A | $R^3$ | $R^1$ | X | $R^2$ | n | Fp (°C) |
|---|---|---|---|---|---|---|---|
| 17.016 | $OCF_2CF_2H$ | H | H | O | $CH_3$ | 1 | 128-130 |
| 17.017 | $OCF_2CF_2H$ | 5-Cl | H | O | $CH_3$ | 0 | |
| 17.018 | $OCHF_2$ | 5-Cl | H | O | $CH_3$ | 0 | |
| 17.019 | $OCF_2CFHCl$ | 5-Cl | H | O | $CH_3$ | 0 | 143-145 Zers. |
| 17.020 | $OCF_2CFHCl$ | H | H | O | $CH_3$ | 1 | |
| 17.021 | $SO_2CH_3$ | 5-Cl | H | O | $CH_3$ | 0 | |
| 17.022 | $SO_2CH_3$ | 5-Cl | H | O | $CH_3$ | 0 | Na-Salz |
| 17.023 | $SO_2CH_3$ | 5-Cl | H | O | $CH_3$ | 1 | |
| 17.024 | $SO_2CH_3$ | 5-Cl | H | O | $CH_3$ | 1 | Na-Salz |
| 17.025 | $SO_2CH_3$ | 6-Cl | H | O | $CH_3$ | 0 | |
| 17.026 | $SO_2CH_3$ | 6-Cl | H | O | $CH_3$ | 0 | Na-Salz |
| 17.027 | $SO_2CH_3$ | 6-Cl | H | O | $CH_3$ | 1 | |
| 17.028 | $SO_2CH_3$ | 6-Cl | H | O | $CH_3$ | 1 | Na-Salz |
| 17.029 | $SO_2CH_3$ | 5-$CH_3$ | H | O | $CH_3$ | 0 | |
| 17.030 | $SO_2CH_3$ | 5-$CH_3$ | H | O | $CH_3$ | 0 | Na-Salz |
| 17.031 | $SO_2CH_3$ | 5-$CH_3$ | H | O | $CH_3$ | 1 | |
| 17.032 | $SO_2CH_3$ | 5-$CH_3$ | H | O | $CH_3$ | 1 | Na-Salz |
| 17.033 | $SO_2CH_3$ | 6-$CH_3$ | H | O | $CH_3$ | 0 | |
| 17.034 | $SO_2CH_3$ | 6-$CH_3$ | H | O | $CH_3$ | 0 | Na-Salz |
| 17.035 | $SO_2CH_3$ | 6-$CH_3$ | H | O | $CH_3$ | 1 | |
| 17.036 | $SO_2CH_3$ | 6-$CH_3$ | H | O | $CH_3$ | 1 | Na-Salz |

Tabelle 17: (Fortsetzung)

| Nr. | A | $R^3$ | $R^1$ | X | $R^2$ | n | Fp (°C) | |
|---|---|---|---|---|---|---|---|---|
| 17.037 | $SO_2CH_3$ | 5-$OCH_3$ | H | O | $CH_3$ | 0 | | |
| 17.038 | $SO_2CH_3$ | 5-$OCH_3$ | H | O | $CH_3$ | 0 | | Na-Salz |
| 17.039 | $SO_2CH_3$ | 5-$OCH_3$ | H | O | $CH_3$ | 1 | | |
| 17.040 | $SO_2CH_3$ | 5-$OCH_3$ | H | O | $CH_3$ | 1 | | Na-Salz |
| 17.041 | $SO_2CH_3$ | 6-$OCH_3$ | H | O | $CH_3$ | 0 | | |
| 17.042 | $SO_2CH_3$ | 6-$OCH_3$ | H | O | $CH_3$ | 0 | | Na-Salz |
| 17.043 | $SO_2CH_3$ | 6-$OCH_3$ | H | O | $CH_3$ | 1 | | |
| 17.045 | $SO_2N(CH_3)_2$ | 5-Cl | H | O | $CH_3$ | 0 | | |
| 17.046 | $SO_2N(CH_3)_2$ | 5-Cl | H | O | $CH_3$ | 0 | | Na-Salz |
| 17.047 | $SO_2N(CH_3)_2$ | 5-Cl | H | O | $CH_3$ | 1 | | |
| 17.048 | $SO_2N(CH_3)_2$ | 5-Cl | H | O | $CH_3$ | 1 | | Na-Salz |
| 17.049 | $SO_2N(CH_3)_2$ | 6-Cl | H | O | $CH_3$ | 0 | | |
| 17.050 | $SO_2N(CH_3)_2$ | 6-Cl | H | O | $CH_3$ | 0 | | Na-Salz |
| 17.051 | $SO_2N(CH_3)_2$ | 6-Cl | H | O | $CH_3$ | 1 | | |
| 17.052 | $SO_2N(CH_3)_2$ | 6-Cl | H | O | $CH_3$ | 1 | | Na-Salz |
| 17.053 | $SO_2N(CH_3)_2$ | 5-$CH_3$ | H | O | $CH_3$ | 0 | | |
| 17.054 | $SO_2N(CH_3)_2$ | 5-$CH_3$ | H | O | $CH_3$ | 0 | | Na-Salz |
| 17.055 | $SO_2N(CH_3)_2$ | 5-$CH_3$ | H | O | $CH_3$ | 1 | | |
| 17.056 | $SO_2N(CH_3)_2$ | 5-$CH_3$ | H | O | $CH_3$ | 1 | | Na-Salz |
| 17.057 | $SO_2N(CH_3)_2$ | 6-$CH_3$ | H | O | $CH_3$ | 0 | | |
| 17.058 | $SO_2N(CH_3)_2$ | 6-$CH_3$ | H | O | $CH_3$ | 0 | | Na-Salz |

Tabelle 17: (Fortsetzung)

| Nr. | A | $R^3$ | $R^1$ | X | $R^2$ | n | Fp (°C) | |
|---|---|---|---|---|---|---|---|---|
| 17.059 | $SO_2N(CH_3)_2$ | 6-$CH_3$ | H | O | $CH_3$ | 1 | | |
| 17.060 | $SO_2N(CH_3)_2$ | 6-$CH_3$ | H | O | $CH_3$ | 1 | | Na-Salz |
| 17.061 | $SO_2N(CH_3)_2$ | 5-$OCH_3$ | H | O | $CH_3$ | 0 | | |
| 17.062 | $SO_2N(CH_3)_2$ | 5-$OCH_3$ | H | O | $CH_3$ | 0 | | Na-Salz |
| 17.063 | $SO_2N(CH_3)_2$ | 5-$OCH_3$ | H | O | $CH_3$ | 1 | | |
| 17.064 | $SO_2N(CH_3)_2$ | 5-$OCH_3$ | H | O | $CH_3$ | 1 | | Na-Salz |
| 17.065 | $SO_2N(CH_3)_2$ | 6-$OCH_3$ | H | O | $CH_3$ | 0 | | |
| 17.066 | $SO_2N(CH_3)_2$ | 6-$OCH_3$ | H | O | $CH_3$ | 0 | | Na-Salz |
| 17.067 | $SO_2N(CH_3)_2$ | 6-$OCH_3$ | H | O | $CH_3$ | 1 | | |
| 17.068 | $SO_2N(CH_3)_2$ | 6-$OCH_3$ | H | O | $CH_3$ | 1 | | Na-Salz |
| 17.069 | $CH_2OCH_3$ | H | H | O | $CH_3$ | 0 | | Na-Salz |
| 17.070 | $CH_2OCH_3$ | H | H | O | $CH_3$ | 1 | | |
| 17.071 | $CH_2OCH_3$ | H | H | O | $CH_3$ | 1 | | Na-Salz |
| 17.072 | $CH=NOCH_3$ | H | H | O | $CH_3$ | 0 | | |
| 17.073 | $CH=NOCH_3$ | H | H | O | $CH_3$ | 0 | | Na-Salz |
| 17.074 | $CH=NOCH_3$ | H | H | O | $CH_3$ | 1 | | |
| 17.075 | $CH=NOCH_3$ | H | H | O | $CH_3$ | 1 | | Na-Salz |
| 17.076 | $CCl_3$ | H | H | O | $CH_3$ | 0 | | |
| 17.077 | $CCl_3$ | H | H | O | $CH_3$ | 0 | | Na-Salz |
| 17.078 | $CCl_3$ | H | H | O | $CH_3$ | 1 | 195 | |
| 17.079 | $CCl_3$ | H | H | O | $CH_3$ | 1 | 125-130 (Zers.) | Na-Salz |

Tabelle 17: (Fortsetzung)

| Nr. | A | $R^3$ | $R^1$ | X | $R^2$ | n | Fp (°C) |
|---|---|---|---|---|---|---|---|
| 17.080 | $OCF_3$ | H | H | O | $CH_3$ | 0 | 127 |
| 17.081 | $OCF_3$ | H | H | O | $CH_3$ | 0 | 119 (Zers.) Na-Salz |
| 17.082 | $OCF_3$ | H | H | O | $CH_3$ | 1 | |
| 17.083 | $OCF_3$ | H | H | O | $CH_3$ | 1 | Na-Salz |
| 17.084 | $CH_2OC_2H_5$ | H | H | O | $CH_3$ | 0 | |
| 17.085 | $CH_2OC_2H_5$ | H | H | O | $CH_3$ | 0 | Na-Salz |
| 17.086 | $CH_2OC_2H_5$ | H | H | O | $CH_3$ | 1 | |
| 17.087 | $CH_2OC_2H_5$ | H | H | O | $CH_3$ | 1 | Na-Salz |
| 17.088 | $O(C=O)CH_3$ | H | H | O | $CH_3$ | 0 | |
| 17.089 | $O(C=O)CH_3$ | H | H | O | $CH_3$ | 0 | Na-Salz |
| 17.090 | $O(C=O)CH_3$ | H | H | O | $CH_3$ | 1 | |
| 17.091 | $O(C=O)CH_3$ | H | H | O | $CH_3$ | 1 | Na-Salz |
| 17.092 | $O(C=O)OCH_3$ | H | H | O | $CH_3$ | 0 | |
| 17.093 | $O(C=O)OCH_3$ | H | H | O | $CH_3$ | 0 | Na-Salz |
| 17.094 | $O(C=O)OCH_3$ | H | H | O | $CH_3$ | 1 | |
| 17.095 | $O(C=O)OCH_3$ | H | H | O | $CH_3$ | 1 | Na-Salz |
| 17.096 | $N(CH_3)COCH_3$ | H | H | O | $CH_3$ | 0 | |
| 17.097 | $N(CH_3)COCH_3$ | H | H | O | $CH_3$ | 0 | Na-Salz |
| 17.098 | $N(CH_3)COCH_3$ | H | H | O | $CH_3$ | 1 | |
| 17.099 | $N(CH_3)COCH_3$ | H | H | O | $CH_3$ | 1 | Na-Salz |
| 17.100 | $N(CH_3)COCH_2Cl$ | H | H | O | $CH_3$ | 0 | |

Tabelle 17: (Fortsetzung)

| Nr. | A | $R^3$ | $R^1$ | X | $R^2$ | n | Fp (°C) | |
|---|---|---|---|---|---|---|---|---|
| 17.101 | $N(CH_3)COCH_2Cl$ | H | H | O | $CH_3$ | 0 | | Na-Salz |
| 17.102 | $N(CH_3)COCH_2Cl$ | H | H | O | $CH_3$ | 1 | | |
| 17.103 | $N(CH_3)COCH_2Cl$ | H | H | O | $CH_3$ | 1 | | Na-Salz |
| 17.104 | $N(CH_3)COCF_3$ | H | H | O | $CH_3$ | 0 | | |
| 17.105 | $N(CH_3)COCF_3$ | H | H | O | $CH_3$ | 0 | | Na-Salz |
| 17.106 | $N(CH_3)COCF_3$ | H | H | O | $CH_3$ | 1 | | |
| 17.107 | $N(CH_3)COCF_3$ | H | H | O | $CH_3$ | 1 | | Na-Salz |
| 17.108 | $N(CH_3)CON(CH_3)_2$ | H | H | O | $CH_3$ | 0 | | |
| 17.109 | $N(CH_3)CON(CH_3)_2$ | H | H | O | $CH_3$ | 0 | | Na-Salz |
| 17.110 | $N(CH_3)CON(CH_3)_2$ | H | H | O | $CH_3$ | 1 | | |
| 17.111 | $N(CH_3)CON(CH_3)_2$ | H | H | O | $CH_3$ | 1 | | Na-Salz |
| 17.112 | $O(C=O)N(CH_3)_2$ | H | H | O | $CH_3$ | 0 | | |
| 17.113 | $O(C=O)N(CH_3)_2$ | H | H | O | $CH_3$ | 0 | | Na-Salz |
| 17.114 | $O(C=O)N(CH_3)_2$ | H | H | O | $CH_3$ | 1 | | |
| 17.115 | $O(C=O)N(CH_3)_2$ | H | H | O | $CH_3$ | 1 | | Na-Salz |
| 17.116 | $N(CH_3)SO_2N(CH_3)_2$ | H | H | O | $CH_3$ | 0 | | |
| 17.117 | $N(CH_3)SO_2N(CH_3)_2$ | H | H | O | $CH_3$ | 0 | | Na-Salz |
| 17.118 | $N(CH_3)SO_2N(CH_3)_2$ | H | H | O | $CH_3$ | 1 | | |
| 17.119 | $N(CH_3)SO_2N(CH_3)_2$ | H | H | O | $CH_3$ | 1 | | Na-Salz |
| 17.120 | $CH_3$ | H | H | O | $CH_3$ | 0 | 161-162 | |
| 17.121 | $CH_3$ | H | H | O | $CH_3$ | 0 | 168 Zers. | Na-Salz |
| 17.122 | $OCH_2CF_3$ | 5-$OCH_2CF_3$ | H | O | $CH_3$ | 0 | 207-210 Zers. | Na-Salz |
| 17.123 | $OCH_2CN$ | H | H | O | $CH_3$ | 0 | 128 Zers. | |
| 17.124 | $OCH_2CN$ | H | H | O | $CH_3$ | 1 | 135 Zers. | |

Anwendungsbeispiele

Die herbizide Wirkung der Sulfonylharnstoffe der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung fördert ein gleichmäßiges Keimen der Testpflanzen, sofern dies nicht durch die Wirkstoffe beeinträchtigt wird.

Zum Zwecke der Nachauflaufbehandlung wurden die Testpflanzen je nach Wuchsform erst bei einer Wuchshöhe von 3 bis 15 cm angezogen und dann mit den in Wasser suspendierten oder emulgierten Wirkstoffen behandelt. Die Aufwandmenge für die Nachauflaufbehandlung betrug 0,5 kg/ha a.S..

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürz. | Lateinischer Name | Deutscher Name |
|---|---|---|
| AMARE | Amaranthus retroflexus L. | Zurückgekrümmter Fuchsschwanz |
| CASTO | Cassia tora L. | Gemüsekassie |
| CENCY | Centaurea cyanus L. | Kornflockenblume |

Mit 0,5 kg a.S./ha im Nachauflaufverfahren eingesetzt, lassen sich mit den Verbindungen 15.005 und 9.011 breitblättrige unerwünschte Pflanzen sehr gut bekämpfen.

Verglichen mit strukturell ähnlichen Verbindungen des Standes der Technik, z.B. EP-A-169 815 weisen die erfindungsgemäßen Verbindungen überraschend vorteilhafte Eigenschaften auf, wie die in nachfolgenden Tabellen I und II zusammengestellten Ergebnisse zeigen. Als Vergleichsmittel A und B dienten folgende Sulfonylharnstoffe:

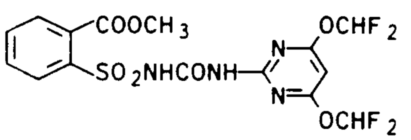

A

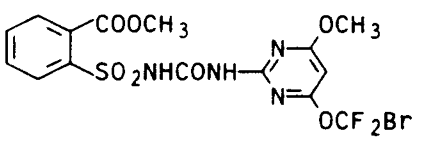

B

Folgene Testpflanzen fanden Verwendung:

| Abkürzung | Lateinischer Name | Deutscher Name |
|---|---|---|
| TRZAW | Triticum aestivum | Winterweizen |
| ABUTH | Abutilon theophrasti | Chinesicher Hanf |
| AMARE | Amaranthus retroflexus | zurückgekrümmter Fuchsschwanz |
| CHEAL | Chenopodium album | weißer Gänsefuß |
| POLPE | Polygonum persicaria | Flohknöterich |
| SINAL | Sinapis alba | Weißer Senf |

Tabelle I

Gegenüberstellung von Ergebnissen aus Gewächshausversuchen im Nachauflaufverfahren

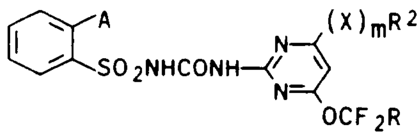

| | | | | |
|---|---|---|---|---|
| Bsp.-Nr. | 9.011 | | A | |
| A | $NO_2$ | | $CO_2CH_3$ | |
| $(X)_mR^2$ | $OCH_3$ | | $OCHF_2$ | |
| R | F | | H | |
| Aufwandmenge (kg/ha a.S.) | 0.06 | 0.03 | 0.06 | 0.03 |
| Testpflanzen | | | | |
| Schädigung in % | | | | |
| TRZAW | 20 | 10 | 70 | 70 |
| ABUTH | 100 | 100 | 100 | 100 |
| AMARE | 100 | 100 | 100 | 100 |
| CHEAL | 100 | 100 | 100 | 100 |
| POLPE | 90 | 90 | 100 | 100 |
| SINAL | 95 | 95 | 95 | 95 |

Tabelle II

Gegenüberstellung von Ergebnissen aus Gewächshausversuchen im Nachauflaufverfahren

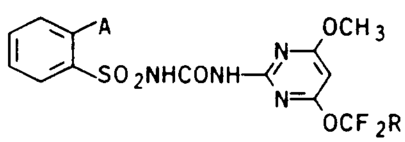

| Bsp.-Nr. | 9.011 | | B | |
|---|---|---|---|---|
| A | $NO_2$ | | $CO_2CH_3$ | |
| R | F | | Br | |
| Aufwandmenge (kg/ha a.S.) | 0.015 | 0.0075 | 0.015 | 0.0075 |
| Testpflanzen (Schädigung in %) | | | | |
| TRZAW | 10 | 0 | 0 | 0 |
| ABUTH | 90 | 85 | 80 | 20 |
| AMARE | 100 | 100 | 50 | 50 |
| CHEAL | 90 | 90 | 50 | 30 |
| POLPE | 70 | 70 | 70 | 50 |
| SINAL | 95 | 90 | 80 | 80 |

**Patentansprüche**

1. Substituierte Sulfonylharnstoffe der allgemeinen Formel I

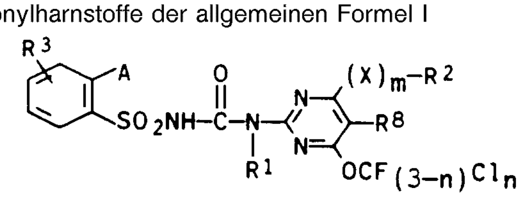

I,

in der n und m für 0 oder 1 stehen und die Substituenten folgende Bedeutung haben:

- $R^1$ Wasserstoff, $C_1$-$C_4$-Alkyl, $C_3$-$C_6$-Alkenyl oder $C_3$-$C_6$-Alkinyl;
- $R^2$ Halogen oder Trifluormethyl, wenn m für 0 steht oder $C_1$-$C_4$-Alkyl, $C_3$-$C_6$-Alkenyl oder $C_3$-$C_6$-Alkinyl, wenn m 1 bedeutet oder Trifluor- oder Chlordifluormethyl, wenn X für 0 oder S und m für 1 stehen;
- X O, S oder N-$R^4$, wobei $R^4$ für Wasserstoff oder $C_1$-$C_4$-Alkyl steht;
- $R^3$ Wasserstoff, Halogen, $C_1$-$C_4$-Alkyl, $C_1$-$C_4$-Halogenalkyl, $C_1$-$C_4$-Alkoxy oder $C_1$-$C_4$-Halogenalkoxy;
- A $NO_2$, $NH_2$, OH, CN, SCN, $S(O)_oR^5$, $SO_2NR^6R^7$, eine Gruppe $ER^7$, in der E für O, S oder $NR^9$ steht,

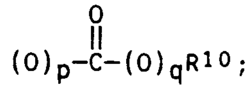

$CH=NOCH_3$;
$C_1$-$C_4$-Alkyl, unsubstituiert oder 1- bis 3-fach substituiert durch Methoxy, Ethoxy, $SO_2CH_3$, Cyano, Rhodano, $SCH_3$;
$C_2$-$C_4$-Alkenyl, unsubstituiert oder 1- bis 3-fach substituiert durch Halogen, Nitro oder Cyano;
- $R^5$ eine $C_1$-$C_6$-Alkylgruppe, welche einen bis drei der folgenden Reste tragen kann: Halogen, $C_1$-$C_4$-Alkoxy, $C_1$-$C_4$-Halogenalkoxy, $C_1$-$C_4$-Alkoxy-$C_1$-$C_2$-alkoxy, $C_3$-$C_7$-Cycloalkyl und/oder

Phenyl; eine $C_5$-$C_7$-Cycloalkylgruppe, welche ein bis drei $C_1$-$C_4$-Alkylgruppen tragen kann; eine $C_2$-$C_6$-Alkenylgruppe oder eine $C_3$-$C_6$-Alkinylgruppe;

$R^6$ Wasserstoff, eine $C_1$-$C_2$-Alkoxygruppe, eine $C_1$-$C_6$-Alkylgruppe, oder gemeinsam mit $R^7$ eine $C_4$-$C_6$-Alkylenkette, worin eine Methylengruppe durch ein Sauerstoffatom oder eine $C_1$-$C_4$-Alkyliminogruppe ersetzt sein kann;

$R^7$ $C_1$-$C_4$-Alkyl, $C_2$-$C_4$-Alkenyl oder $C_3$-$C_4$-Alkinyl, wobei die genannten Reste noch ein bis vier Halogen- oder $C_1$-$C_4$-Alkoxyreste tragen können; eine $C_3$-$C_6$-Cycloalkylgruppe; ferner im Fall von E = $NR^9$ noch Methylsulfon, Trifluormethylsulfon, Ethylsulfon, Acetyl, das ein bis drei Halogenatome tragen kann, Methoxycarbonyl, Dimethylcarbamoyl oder Dimethylsulfamoyl;

o 0, 1 oder 2;

p,q 0 und/oder 1, wobei im Falle von p = 0 q ebenfalls für 0 steht;

$R^8$ Wasserstoff oder Halogen;

$R^9$ Wasserstoff, Methyl oder Ethyl;

$R^{10}$ $C_1$-$C_3$-Alkyl, $C_1$-$C_3$-Halogenalkyl, $C_1$-$C_2$-Alkoxy-$C_1$-C2-alkyl, $C_2$-$C_4$-Alkenyl, $C_3$-$C_6$-Cycloalkyl, $C_2$-$C_3$-Halogenalkenyl, ferner im Fall von p = 1 und q = 0 noch $C_1$-$C_3$-Alkylamino oder Di-($C_1$-$C_2$-Alkyl)amino

sowie deren umweltverträgliche Salze.

**2.** Sulfonylharnstoffe der Formel I nach Anspruch 1, in der die Substituenten folgende Bedeutung haben:

$R^1$ Wasserstoff oder Methyl;

$R^2$ Halogen oder Trifluormethyl, wenn m für 0 steht und Methyl, wenn m 1 bedeutet;

X O oder NH;

$R^3$ Wasserstoff, Halogen, Methyl oder Methoxy;

A $NO_2$, $N[CH_3]SO_2CH_3$, eine Gruppe $SO_2R^5$, worin $R^5$ $C_1$-$C_4$-Alkyl bedeutet, eine Gruppe $SO_2NR^6R^7$, worin $R^6$ und $R^7$ Methyl bedeuten, eine $OR^7$-Gruppe, worin $R^7$ $C_1$-$C_2$-Alkyl, das ein bis drei bzw. vier Halogenatome oder eine Methoxygruppe tragen kann, bedeutet;

$R^8$ Wasserstoff;

sowie deren umweltverträgliche Salze.

**3.** Herbizides Mittel, enthaltend einen Sulfonylharnstoff der Formel I gemäß Anspruch 1 oder sein Salz sowie hierfür übliche Trägerstoffe.

**4.** Verfahren zur Bekämpfung unerwünschten Pflanzenwuchses, dadurch gekennzeichnet, daß man einen Sulfonylharnstoff der Formel I gemäß Anspruch oder eines seiner Salze in einer herbizid wirksamen Menge auf die Pflanzen und/oder ihren Lebensraum einwirken läßt.

**5.** Verfahren zur Herstellung von Sulfonylharnstoffen der Formel I gemäß Anspruch 1, dadurch gekennzeichnet, daß man ein Sulfonylisocyanat II

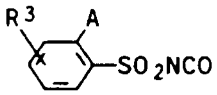

II

in an sich bekannter Weise in einem inerten organischen Lösungsmittel mit der ungefähr stöchiometrischen Menge eines substituierten 2-Amino-4-fluoralkoxy-pyrimidins der Formel III

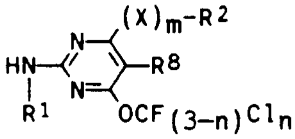

III

umsetzt.

**6.** Verfahren zur Herstellung der Verbindungen I gemäß Anspruch 1, dadurch gekennzeichnet, daß man ein Carbamat der Formel IV

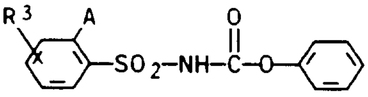 IV

in an sich bekannter Weise in einem inerten organischen Lösungsmittel bei einer Temperatur zwischen 0 und 120 °C mit ungefähr der stöchiometrischen Menge eines 2-Amino-4-fluoralkoxy-pyrimidinsIII umsetzt.

**7.** Verfahren zur Herstellung von Sulfonylharnstoffen der Formel I gemäß Anspruch 1, dadurch gekennzeichnet, daß man ein entsprechendes Sulfonamid der Formel V

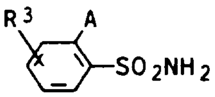 V

in an sich bekannter Weise in einem inerten organischen Lösungsmittel mit einem Phenylcarbamat VI

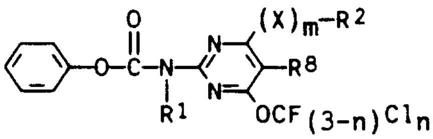 VI

umsetzt.

**8.** Verfahren zur Herstellung von Sulfonylharnstoffen der Formel I gemäß Anspruch 1, in der A für eine Gruppe $NR^7R^9$ oder eine Gruppe $O-(C=O)-(O)_qR^{10}$ und $R^7$ für Methylsulfon, Ethylsulfon, Trifluormethylsulfon, Acetyl, das bis zu 3 Halogenatome tragen kann, Methoxycarbonyl, Dimethylcarbamoyl oder Dimethylsulfamoyl steht und $R^9$ und $R^{10}$ die im Anspruch 1 genannte Bedeutung haben, dadurch gekennzeichnet, daß man einen Sulfonylharnstoff der Formel VIa

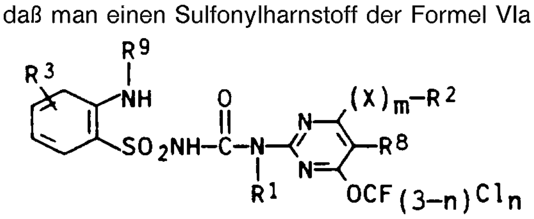 VIa

mit einem Sulfonierungs- oder Acylierungsagens der Formel VIb

$R^7$-L    VIb

bzw. einen Sulfonylharnstoff der Formel VIc

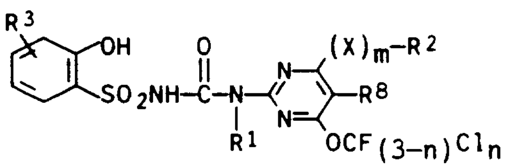 VIc

mit einem Acylierungsagens der Formel VId

$R^{10}-(O)_q(C=O)-L$    VId,

wobei $R^7$, $R^{10}$ und q die oben genannte Bedeutung haben und L für eine Abgangsgruppe wie ein

Halogenatom, eine Carboxylatgruppe oder eine Sulfonatgruppe steht, in an sich bekannter Weise in einem inerten organischen Lösungsmittel bei einer Temperatur zwischen 0 und 120 ° C umsetzt.

## Claims

**1.** A substituted sulfonylurea of formula I

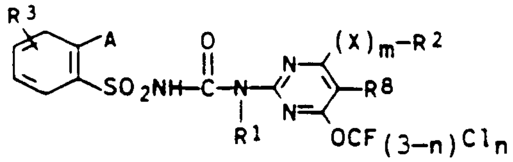

I,

where n and m are each 0 or 1;

$R^1$ is hydrogen, $C_1$-$C_4$-alkyl, $C_3$-$C_6$-alkenyl or $C_3$-$C_6$-alkynyl; $R^2$ is halogen or trifluoromethyl when m is 0 or $C_1$-$C_4$-alkyl, $C_3$-$C_6$-alkenyl or $C_3$-$C_6$-alkynyl when m is 1 or trifluoro- or chlorodifluoromethyl when X is O or S and m is 1; X is O, S or N-$R^4$, where $R^4$ is hydrogen or $C_1$-$C_4$-alkyl; $R^3$ is hydrogen, halogen, $C_1$-$C_4$-alkyl, $C_1$-$C_4$-haloalkyl, $C_1$-$C_4$-alkoxy or $C_1$-$C_4$-haloalkoxy;

A is $NO_2$, $NH_2$, OH, CN, SCN, $S(O)_oR^5$, $SO_2NR^6R^7$, a group $ER^7$, where E is O, S or $NR^9$,

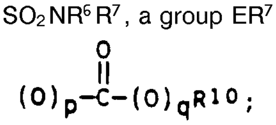

;

$CH=NOCH_3$;

$C_1$-$C_4$-alkyl which is unsubstituted or mono-, di- or trisubstituted by methoxy, ethoxy, $SO_2CH_3$, cyano, thiocyanato or $SCH_3$, or $C_2$-$C_4$-alkenyl which is unsubstituted or mono-, di- or trisubstituted by halogen, nitro or cyano;

$R^5$ is $C_1$-$C_6$-alkyl which may carry from one to three of the following radicals: halogen, $C_1$-$C_4$-alkoxy, $C_1$-$C_4$-haloalkoxy, $C_1$-$C_4$-alkoxy-$C_1$- or $C_2$-alkoxy, $C_3$-$C_7$-cycloalkyl and/or phenyl; $C_5$-$C_7$-cycloalkyl which may carry from one to three $C_1$-$C_4$-alkyl groups; $C_2$-$C_6$-alkenyl or $C_3$-$C_6$-alkynyl;

$R^6$ is hydrogen, $C_1$- or $C_2$-alkoxy or $C_1$-$C_6$-alkyl, or, together with $R^7$, forms a $C_4$-$C_6$-alkylene chain in which a methylene group may be replaced with an oxygen atom or a $C_1$-$C_4$-alkylimino group;

$R^7$ is $C_1$-$C_4$-alkyl, $C_2$-$C_4$-alkenyl or $C_3$- or $C_4$-alkynyl, it being possible for the stated radicals to carry a further one to four halogen or $C_1$-$C_4$-alkoxy radicals, or is $C_3$-$C_6$-cycloalkyl, or, where E is $NR^9$, is furthermore methylsulfonyl, trifluoromethylsulfonyl, ethylsulfonyl, acetyl which may carry one to three halogen atoms, or methoxycarbonyl, dimethylcarbamoyl or dimethylsulfamoyl;

o is 0, 1 or 2;

p and q are 0 or 1, and, where p is 0, q is likewise 0, and

$R^8$ is hydrogen or halogen;

$R^9$ is hydrogen, methyl or ethyl;

$R^{10}$ is $C_1$-$C_3$-alkyl, $C_1$-$C_3$-haloalkyl, $C_1$- or $C_2$-alkoxy-$C_1$- or $C_2$-alkyl, $C_2$-$C_4$-alkenyl, $C_3$-$C_6$-cycloalkyl or $C_2$- or $C_3$-haloalkenyl, or, where p is 1 and q is 0, is furthermore $C_1$-$C_3$-alkylamino or di-($C_1$- or $C_2$-alkyl)-amino,

and environmentally compatible salts thereof.

**2.** A sulfonylurea of the formula I as claimed in claim 1,
where

| | |
|---|---|
| $R^1$ | is hydrogen or methyl; |
| $R^2$ | is halogen or trifluoromethyl when m is 0 and methyl when m is 1; |
| X | is O or NH; |
| $R^3$ | is hydrogen, halogen, methyl or methoxy; |
| A | is $NO_2$, $N[CH_3]SO_2CH_3$, a group $SO_2R^5$, where $R^5$ is $C_1$-$C_4$-alkyl, a group $SO_2NR^6R^7$, where $R^6$ and $R^7$ are each methyl, or an $OR^7$ group, where $R^7$ is $C_1$- or $C_2$-alkyl which may carry from one to three or four halogen atoms or one methoxy group; |

and

| | |
|---|---|
| $R^8$ | is hydrogen, |

and environmentally compatible salts thereof.

3. Herbicide containing a sulfonylurea of the formula I as claimed in claim 1 or a salt and carriers conventionally used for this purpose.

4. A method for controlling undesirable plant growth, wherein a sulfonylurea of the formula I as claimed in claim 1 or one of its salts is allowed to act, in a herbicidally effective amount, on the plants or their habitat.

5. A process for the preparation of a sulfonylurea of the formula I as claimed in claim 1, wherein a sulfonyl isocyanate II

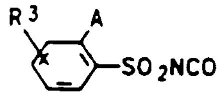

II

is reacted in a conventional manner, in an inert organic solvent, with about the stoichiometric amount of a substituted 2-amino-4-fluoroalkoxypyrimidine of the formula III

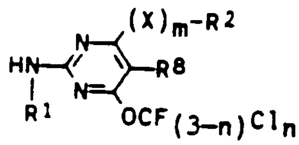

III

6. A process for the preparation of a compound I as claimed in claim 1, wherein a carbamate of the formula IV

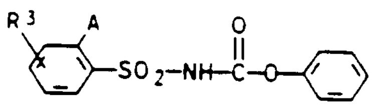

IV

is reacted in a conventional manner, in an inert organic solvent at from 0 to 120 °C, with about the stoichiometric amount of a 2-amino-4-fluoroalkoxypyrimidine III.

7. A process for the preparation of a sulfonylurea of the formula I as claimed in claim 1, wherein a corresponding sulfonamide of the formula V

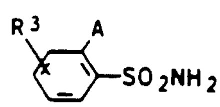

V

is reacted in a conventional manner, in an inert organic solvent, with a phenyl carbamate VI

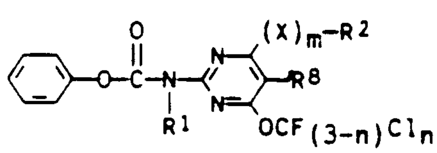

VI

8. A process for the preparation of a sulfonylurea of the formula I as claimed in claim 1, where A is a group $NR^7R^9$ or a group $O\text{-}(C=O)\text{-}(O)_qR^{10}$ and $R^7$ is methylsulfonyl, ethylsulfonyl, trifluoromethylsulfonyl, acetyl which may carry up to 3 halogen atoms, or methoxycarbonyl, dimethylcarbamoyl or dimethylsulfamoyl and $R^9$ and $R^{10}$ each have the meanings stated in claim 1, wherein a sulfonylurea of

the formula VIa

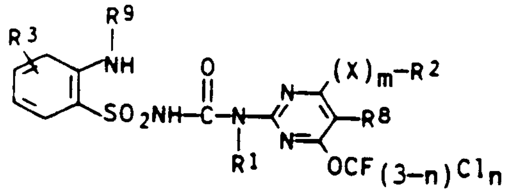

VIa

is reacted with a sulfonation or acylation agent of the formula VIb

$R^7$-L VIb

or a sulfonylurea of the formula VIc

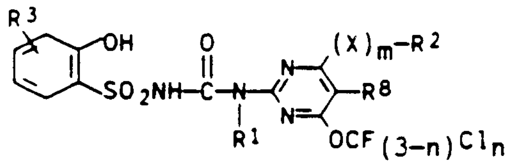

VIc

is reacted with an acylating agent of the formula VId

$R^{10}$-$(O)_q$(C = O)-L VId,

where $R^7$, $R^{10}$ and q have the abovementioned meanings and L is a leaving group, such as halogen, carboxylate or sulfonate, in a conventional manner in an inert organic solvent at from 0 to 120 ° C.

## Revendications

**1.** Sulfonylurées substitués de la formule générale I

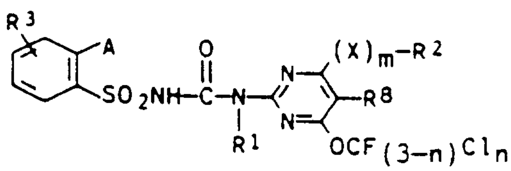

I,

dans laquelle n et m sont mis pour 0 ou 1 et les substituants ont la signification suivante :

- $R^1$ hydrogène, alkyle en C1-C4, alcényle en C3-C6 ou alcynyle en C3-C6 ;
- $R^2$ halogène ou trifluorométhyle quand m est mis pour O ou alkyle en C1-C4, alcényle en C3-C6 ou alcynyle en C3-C6 quand m est mis pour 1, ou trifluoro- ou chlorodifluorométhyle quand X est mis pour O ou S et m pour 1 ;
- X O, S ou N-$R^4$, $R^4$ représentant hydrogène ou alkyle en C1-C4 ;
- $R^3$ hydrogène, halogène, alkyle en C1-C4, halogénoalkyle en C1-C4, alcoxy en C1-C4 ou halogénoalcoxy en C1-C4 ;
- A $NO_2$, $NH_2$, OH, CN, SCN, $S(O)_oR^5$, $SO_2NR^6R^7$, un groupe $ER^7$, dans lequel E est mis pour O, S ou $NR^9$,

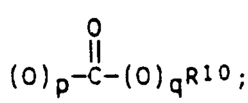

$CH=NOCH_3$;
alkyle en C1-C4, non substitué ou substitué 1 à 3 fois par méthoxy, éthoxy, $SO_2CH_3$, cyano, rhodano, $SCH_3$ ;
alcényle en C2-C4, non substitué ou substitué 1 à 3 fois par halogène, nitro ou cyano ;

$R^5$ un groupe alkyle en C1-C6, qui peut porter un à trois des restes suivants : halogène, alcoxy en C1-C4, halogénalcoxy en C1-C4, alcoxy en C1-C4-alcoxy en C1-C2, cycloalkyle en C3-C7 et/ou phényle ; un groupe cycloalkyle en C5-C7, qui peut porter un à trois groupes alkyle en C1-C4; un groupe alcényle en C2-C6 ou un groupe alcynyle en C3-C6 ;

$R^6$ hydrogène, un groupe alcoxy en C1-C2, un groupe alkyle en C4-C6 ou, avec $R^7$, une chaîne alkylène en C4-C6, où un groupe méthylène peut être remplacé par un atome d'oxygène ou un groupe (alkyle en C1-C4)-imino ;

$R^7$ alkyle en C1-C4, alcényle en C2-C4 ou alcynyle en C3-C4, les restes indiquées pouvant encore porter un à quatre restes halogène ou alcoxy en C1-C4 ; un groupe cycloalkyle en C3-C6, en outre, lorsque $E = NR^9$, encore méthylsulfone, trifluorométhylsulfone, éthylsulfone, acétyle, qui peut porter trois atomes d'halogène, méthoxycarbonyle, diméthylcarbamoyle ou diméthylsulfamoyle ;

o 0, 1 ou 2 ;

p, q 0 et/ou 1, et dans le cas où p = 0, q est mis aussi pour 0 ;

$R^8$ hydrogène ou halogène ;

$R^9$ hydrogène, méthyle ou éthyle ;

$R^{10}$ alkyle en C1-C3, halogénalkyle en C1-C3, alcoxy en C1-C2-alkyle en C1-C2, alcényle en C2-C4, cycloalkyle en C3-C6 halogénalcényle en C2-C3, en outre si p = 1 et q = 0 encore (alkyle en C1-C3)-amino ou di-(alkyle en C1-C2)-amino

ainsi que leurs sels acceptables pour l'environnement.

**2.** Sulfonylurées de formule I selon la revendication 1, dans laquelle les substituants ont la signification suivante :

$R^1$ hydrogène ou méthyle ;

$R^2$ halogène ou trifluorométhyle, quand m est mis pour 0 et méthyle quand m représente 1 ;

X O ou NH ;

$R^3$ hydrogène, halogène, méthyle ou méthoxy ;

A $-NO_2$, $N(CH_3)SO_2CH_3$, un groupe $SO_2R^5$, où $R^5$ représente alkyle en C3-C4, un groupe $SO_2NR^6R^7$, où $R^6$ et $R^7$ représentent méthyle, un groupe $OR^7$, où $R^7$ représente alkyle en C1-C2 qui peut porter un à trois ou quatre atomes d'halogène ou un groupe méthoxy ;

$R^8$ hydrogène ;

ainsi que leurs sels acceptables pour l'environnement.

**3.** Agent herbicide contenant une sulfonylurée de formule I selon la revendication 1 ou son sel ainsi que des supports usuels pour cela.

**4.** Procédé de lutte contre une croissance indésirable de plantes, caractérisé par le fait que l'on fait agir une sulfonylurée de formule I selon la revendication 1 ou un de ses sels, en quantité active comme herbicide, sur les plantes et/ou leur biotope.

**5.** Procédé de préparation de sulfonylurées de formule I selon la revendication 1, caractérisé par le fait que l'on fait agir un sulfonylisocyanate II

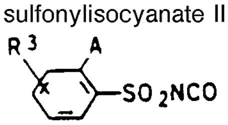

II

de manière connue en soi, dans un solvant organique inerte, avec une quantité à peu près stoechiométrique d'une 2-amino-4-fluoro-alcoxy-pyrimidine de la formule III

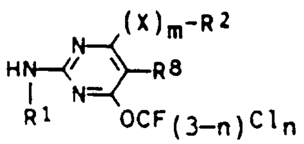

III

6. Procédé de préparation de composés I selon la revendication 1, caractérisé par le fait que l'on fait réagir un carbamate de formule IV

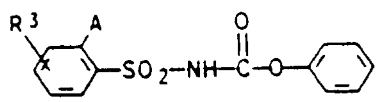

IV

de manière connue en soi, dans un solvant organique inerte, à une température comprise entre 0 et 120 ° C, avec une quantité à peu près stoechiométrique d'une 2-amino-4-fluoro-alcoxy-pyrimidine III.

7. Procédé de préparation de sulfonylurées de formule I selon la revendication 1, caractérisé par le fait que l'on fait réagir une sulfonamide correspondant de formule V

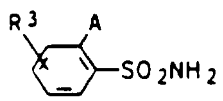

V

de manière connue en soi, dans un solvant organique inerte, avec un phénylcarbamate VI

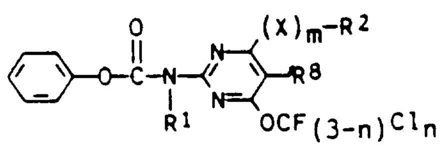

VI

8. Procédé de préparation de sulfonylurées de formule I selon la revendication 1, dans laquelle A est mis pour un groupe $NR^7R^9$ ou un groupe $O\text{-}(C=O)\text{-}O)_qR^{10}$ et $R^7$ pour méthylsulfone, éthylsulfone, trifluorométhylsulfone, acétyle, qui peut porter jusqu'à 3 atomes d'halogène, méthoxycarbonyle, diméthylcarbamoyle ou diméthylsulfamoyle et $R^9$ et $R^{10}$ ont la signification indiquée dans la revendication 1, caractérisé par le fait que l'on fait réagir, de manière connue en soi, dans un solvant organique inerte, à une température comprise entre 0 et 120 ° C, une sulfonylurée de formule VIa

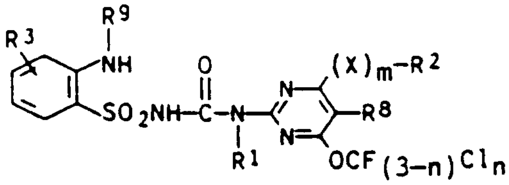

VIa

avec un agent de sulfonation ou acylation de formule VIb

R7-L VIb

ou une sulfonyurée de formule VIc

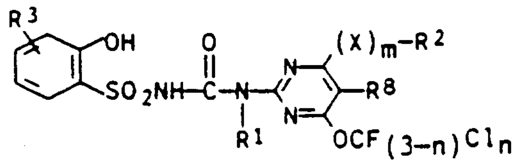

VIc

avec un agent d'acylation de formule VId

$R^{10}$-$(O)_q$(C = O)-L VId,

$R^7$, $R^{10}$ et q ayant la signification indiquée ci-dessus et L étant mis pour un groupe éliminable tel qu'un atome d'halogène, un groupe carboxylate ou un groupe sulfonate.